# EUROPEAN PATENT APPLICATION

(11) **EP 4 206 218 A1**
(43) Date of publication of application: **05.07.2023**
(21) Application number: 21929756.1
(22) Date of filing: 17.06.2021
(51) Int. Cl.: C07K 14/415, C12N 15/29, C12N 15/82, A01H 5/10, A01H 1/04, C12Q 1/6895

(54) **PROTEIN AND BIOMATERIAL RELATED TO RICE YIELD AND APPLICATION OF BOTH IN IMPROVING RICE YIELD**

(30) Priority: 10.03.2021 CN 202110259877; 02.04.2021 CN 202110360014
(71) Applicant: Institute of Crop Sciences, Chinese Academy of Agricultural Sciences, Beijing 100081 (CN)
(72) Inventor: ZHOU, Wenbin, Beijing 100081 (CN); LI, Xia, Beijing 100081 (CN); WEI, Shaobo, Beijing 100081 (CN)
(74) Representative: Fuchs Patentanwälte Partnerschaft mbB
(86) International application number: PCT/CN2021/100542
(87) International publication number: WO 2022/188286

(57) **Abstract**

The invention discloses proteins and biological materials related to rice *(Oryza sativa* L.) yield, and use thereof in increasing rice yield. The protein related to rice yield disclosed by the invention is OsDREB1C having SEQ ID NO: 1 in the Sequence Listing as its sequence, and having SEQ ID NO: 2 in the Sequence Listing as its coding gene sequence. Experiments have demonstrated that OsDREB1C and the associated biological materials thereof of the invention can enhance the photosynthetic efficiency of a plant, promote nitrogen uptake and transport and increase the nitrogen content in the plant and in its grains, promote earlier heading, and improve yield. The OsDREB1C and the associated biological materials thereof of the invention are of great biological significance and industrial value, and find bright prospects for application. In addition, the haplotype Hap.3 discovered by the invention based on OsDREB1C has excellent traits that increase yield, and finds bright prospects in application to genetic breeding of rice and cultivar modification.

## Description

### TECHNICAL FIELD

The invention relates to proteins and biological materials related to rice *(Oryza sativa* L.) yield, and use thereof in rice yield increase in the field of biotechnology.

### BACKGROUND

With a growth in population and economic development in future, China's food demand will continue to grow rapidly. In such a situation where reduction in cultivated land continues, and the potential for expanding area for growing food crops is to be constrained, continuing to improving crop yield per unit area in a larger area has become the only choice to ensure China's food security. Therefore, under the pressure of food security demand, high yield is the unremitting goal for agricultural production to reach. The "Green Revolution", which surged in the middle of 1950s, has achieved a substantial leap in the potential of crop yield through genetic modification of crop varieties and enhancement of cultivation and management techniques. In recent years, however, crop yield per unit area has stagnated or even declined. How to promote crop yield per unit area requires new strategies and approaches.

Heading stage, as one of the important agronomic traits of crops, determines the season, regional adaptability and yield of rice. A proper heading stage leads to a stable and high yield of crops. Selecting and breeding new cultivars with prematurity and high yield has always been one of the most primary focuses in research of crop genetics and breeding. "High yield but without prematurity, prematurity but without high yield" - a phenomenon described as "excellent but not early, early but not excellent", is a hard nut to crack to cultivate crops' strains.

In agricultural production, application of nitrogen fertilizer in a vast amount has always been one of the important measures for high-yield of crops. A continuing, large amount of nitrogen fertilizer input not only increases cost for planting, but also leads to increasingly serious environmental pollution. How to lower the input of nitrogen fertilizer in agricultural production and persistently improve crop yield has become a significant concern in dire need of being settled for the sustainable development of current agriculture in China. It is an urgent task for the sustainable development of agriculture in China to explore the regulation mechanism and technical routes for a substantial boost in crop yield and efficient harness of resources.

At present, researchers who have been carried out relevant exploration and research in crop breeding, cultivation and management measures, and functional gene discovering made some important progress. However, there is not an efficient solution for two major issues yet: high yield with high resource efficiency, and high yield with prematurity in crop production as mentioned above.

In agricultural production, growth phase of crops plays a decisive role in crop yield, among which heading stage of rice is one of the most important agronomic traits that determine the yield and quality of rice. Appropriate heading stage plays a key role in adapting rice varieties to different ecological regions and different planting seasons, and is the guarantee of stable and high yield of crops. For long time, selecting and breeding new cultivars with prematurity and high yield has always been one of the most primary focuses in research of crop genetics and breeding. Primary influencing factors responsible for heading stage include variety differences, photoperiod, and cultivation fashions, etc. Different heading stages of different rice cultivars directly affect cultivated region, seasonal adaptability, and yield traits of cultivars. Therefore, exploring and identifying candidate genes and molecular markers related to heading stage and yield from different rice germplasm resources is very necessary for the genetic modification of rice varieties. Heading stage and yield of rice, as typical, complex quantitative traits, are controlled by multiple genes. Usually, there are multiple SNP loci in a specific candidate gene, and the phenotypic traits are not caused by a single SNP, but by a specific combination of multiple SNPs. The specific combination of such kind of SNPs with statistical correlation is haplotype. Haplotype analysis of SNP information in a large number of rice germplasm materials can provide more extended knowledge for study of candidate genes in charge of rice heading stage and yield, and also provide a more reliable reference for directional selection in the process of natural selection and artificial domestication.

### SUMMARY

One technical problem to be solved by the present invention is: how to increase yield of a plant.

In order to solve the above technical problem, the present invention first provides any of the following uses of a protein, or a material that regulates the activity or content of the protein in:
D1) regulation of yield of a plant;
D2) preparation of a product that regulates yield of a plant;
D3) regulation of photosynthesis and yield of a plant;
D4) preparation of a product that regulates photosynthesis and yield of plant;
D5) regulation of photosynthesis, nitrogen uptake or transport in, and yield of a plant;
D6) preparation of a product that regulates nitrogen uptake or transport in, photosynthesis and yield of a plant;
D7) regulation of nitrogen content in, photosynthesis and yield of a plant;
D8) preparation of a product that regulates nitrogen content in, photosynthesis and yield of a plant;
D9) regulation of nitrogen uptake or transport in, photosynthesis, blooming stage, and yield of a plant;
D10) preparation of a product that regulates nitrogen uptake or transport in, photosynthesis, blooming stage, and yield of a plant;
D11) regulation of nitrogen content in, photosynthesis, blooming stage, and yield of a plant;
D12) preparation of a product that regulates nitrogen content in, photosynthesis, blooming stage, and yield of a plant;
D13) breeding of a plant;
the protein (referred to as OsDREB 1C) is A1), A2), A3) or A4), as follows:
A1) a protein having an amino acid sequence of SEQ ID NO: 1;
A2) a protein obtained by subjecting the amino acid sequence shown in SEQ ID NO: 1 in the Sequence Listing to substitution and/or deletion and/or addition of one or more amino acid residues and having the same function;
A3) a protein derived from rice *(Oryza sativa* L.), millet (*Setaria italica*), maize (*Zea mays*), sorghum (*Sorghum bicolor*), aegilops (*Aegilops tauschii*), wheat *(Triticum aestivum),* or *Brachypodium distachyon* and having 64% or more identity with SEQ ID NO: 1 and having the same function as the protein described in A1);
A4) a fusion protein obtained by linking a tag to the N-terminus or/and C-terminus of A1) or A2) or A3).

To facilitate the purification of the protein in A1), a tag as shown in the table below may be linked to the amino terminus or the carboxyl terminus of the protein consisting of the amino acid sequence shown in SEQ ID NO: 1 in the Sequence Listing.

**Table. Sequence of the Tag**

| Tag | Residues | Sequence |
|---|---|---|
| Poly-Arg | 5-6 (usually 5) | RRRRR |
| Poly-His | 2-10 (usually 6) | HHHHHH |
| FLAG | 8 | DYKDDDDK |
| Strep-tag II | 8 | WSHPQFEK |
| c-myc | 10 | EQKLISEEDL |

The protein in A2) above is a protein having 64% or more identity with, and the same function as the amino acid sequence of the protein shown in SEQ ID NO: 1. Said having 64% or more identity is having 64%, having 75%, having 80%, having 85%, having 90%, having 95%, having 96%, having 97%, having 98%, or having 99% identity.

The protein in A2) above can be artificially synthesized, or can be obtained by synthesizing the coding gene thereof first and then performing biological expression.

The coding gene of the protein in A2) above can be obtained by: deleting codon(s) of one or more amino acid residues, and/or performing missense mutations of one or more base pairs, and/or linking the coding sequence of the tag shown in the table above to the 5'-terminal and/or 3'-terminal, in the DNA sequence shown in SEQ ID NO: 2, in which the DNA molecule shown in SEQ ID NO: 2 encodes the protein shown in SEQ ID NO: 1.

The invention further provides any of the following uses of a biological material associated with OsDREB1C in:
D1) regulation of yield of a plant;
D2) preparation of a product that regulates yield of a plant;
D3) regulation of photosynthesis and yield of a plant;
D4) preparation of a product that regulates photosynthesis and yield of plant;
D5) regulation of photosynthesis of, nitrogen uptake or transport in, and yield of a plant;
D6) preparation of a product that regulates photosynthesis of, nitrogen uptake or transport in, and yield of a plant;
D7) regulation of photosynthesis of, nitrogen content in, and yield of a plant;
D8) preparation of a product that regulates photosynthesis of, nitrogen content in, and yield of a plant;
D9) regulation of photosynthesis of, nitrogen uptake or transport in, blooming stage of, and yield of a plant;
D10) preparation of a product that regulates photosynthesis of, nitrogen uptake or transport in, blooming stage of, and yield of a plant;
D11) regulation of nitrogen content in, photosynthesis of, blooming stage of, and yield of a plant;
D12) preparation of a product that regulates nitrogen content in, photosynthesis of, blooming stage of, and yield of a plant;
D13) breeding of a plant;
the biological material is any of B1) to B9), as follows:
B1) a nucleic acid molecule encoding OsDREB1C;
B2) an expression cassette containing the nucleic acid molecule described in B1);
B3) a recombinant vector containing the nucleic acid molecule described in B1), or a recombinant vector containing the expression cassette described in B2);
B4) a recombinant microorganism containing the nucleic acid molecule described in B1), or a recombinant microorganism containing the expression cassette described in B2), or a recombinant microorganism containing the recombinant vector described in B3);
B5) a transgenic plant cell line containing the nucleic acid molecule described in B1), or a transgenic plant cell line containing the expression cassette described in B2);
B6) a transgenic plant tissue containing the nucleic acid molecule described in B1), or a transgenic plant tissue containing the expression cassette described in B2);
B7) a transgenic plant organ containing the nucleic acid molecule described in B1), or a transgenic plant organ containing the expression cassette described in B2);
B8) a nucleic acid molecule for reducing the expression of OsDREB1C, or with OsDREB1C coding gene knocked out;
B9) an expression cassette, a recombinant vector, a recombinant microorganism, a transgenic plant cell line, a transgenic plant tissue, or a transgenic plant organ containing the nucleic acid molecule described in B8).

Among the above uses, the nucleic acid molecule described in B1) can be b11) or b12) or b13) or b14) or b15), as follows:
b11) a cDNA or DNA molecule having a coding sequence of SEQ ID NO: 2 in the Sequence Listing;
b12) a cDNA or DNA molecule shown in SEQ ID NO: 2 in the Sequence Listing;
b13) a DNA molecule shown in SEQ ID NO: 3 at positions 3001-4131 in the Sequence Listing;
b14) a cDNA or DNA molecule having 73% or more identity with the nucleotide sequence defined in b11) or b12) or b13), and encoding OsDREB 1C;
b15) a cDNA or DNA molecule hybridizing with the nucleotide sequence defined in b11) or b12) or b13) or b14) under stringent conditions, and encoding OsDREB 1C;
B2) the expression cassette is b21) or b22) or b23), as follows:
   b21) a DNA molecule shown in SEQ ID NO: 3 in the Sequence Listing;
   b22) a DNA molecule having 73% or more identity with, and the same function as the nucleotide sequence defined in b21);
   b23) a DNA molecule hybridizing, under stringent conditions, with and having the same function as the nucleotide sequence defined in b21) or b22),
   wherein, the nucleic acid molecule can be DNA, such as cDNA, genomic DNA, or recombinant DNA; the nucleic acid molecule can also be RNA, such as mRNA, or hnRNA, and the like.

The nucleotide sequence encoding the protein OsDREB1C of the invention may be readily mutated by the skilled in the art using known methods, such as directed evolution and point mutation. Those artificially modified nucleotides having 73% or more identity with the nucleotide sequence of the protein OsDREB1C as isolated in the invention are all derived from the nucleotide sequence of the invention and equivalent to the sequence of the invention, with the proviso that they encode the protein OsDREB1C, and have the function thereof.

The term "identity", as used herein, refers to the sequence similarity to a native nucleic acid sequence. "Identity" comprises nucleotide sequences having 73% or more, or 85% or more, or 90% or more, or 95% or more identity with the nucleotide sequence of the invention encoding the protein consisting of the amino acid sequence shown in SEQ ID NO: 1. Identity can be evaluated with naked eyes or computer software. Using the computer software, the identity between two or more sequences can be expressed in percentage (%), which can be used to evaluate the identity between related sequences.

Among the uses above, the stringent conditions can be as follows: hybridizing at 50°C in a mixed solution of 7% sodium dodecyl sulfate (SDS), 0.5M NaPO₄, and 1mM EDTA, and rinsing at 50°C in 2 × SSC, 0.1% SDS; or, as follows: hybridizing at 50°C in a mixed solution of 7% SDS, 0.5M NaPO₄, and 1mM EDTA, and rinsing at 50°C in 1 × SSC, 0.1% SDS; or, as follows: hybridizing at 50°C in a mixed solution of 7% SDS, 0.5M NaPO₄, and 1mM EDTA, and rinsing at 50°C in 0.5 × SSC, 0.1% SDS; or, as follows: hybridizing at 50°C in a mixed solution of 7% SDS, 0.5M NaPO₄, and 1mM EDTA, and rinsing at 50°C in 0.1 × SSC, 0.1% SDS; or, as follows: hybridizing at 50°C in a mixed solution of 7% SDS, 0.5M NaPO₄, and 1mM EDTA, and rinsing at 65°C in 0.1 × SSC, 0.1% SDS; or, as follows: hybridizing at 65°C in a solution of 6 × SSC, 0.5% SDS, and then washing membrane once with 2 × SSC, 0.1% SDS, and 1 × SSC, 0.1% SDS, respectively; or, as follows: hybridizing at 68°C in a solution of 2 × SSC, 0.1% SDS and washing membrane twice, each for 5min, and then hybridizing at 68°C in a solution of 0.5 × SSC, 0.1% SDS and washing membrane twice, each for 15min; or, as follows: hybridizing at 65°C in a solution of 0.1 × SSPE (or 0.1× SSC), 0.1% SDS, and washing membrane.

The 73% or more identity described above can be 80%, 85%, 90%, or 95% or more identity.

Among the uses above, the expression cassette containing a nucleic acid molecule encoding the OsDREB1C protein (OsDREB1C gene expression cassette) described in B2) refers to the DNA that can enables expression of the OsDREB 1C protein in host cells. This DNA can include not only a promoter that initiates OsDREB1C gene transcription, but also a terminator that terminates OsDREB1C gene transcription. Further, the expression cassette may include an enhancer sequence. Promoters that can be used in the invention include, but are not limited to, constitutive promoters, tissue-, organ-, and development- specific promoters, and inducible promoters. Examples of promoters include, but are not limited to: constitutive promoter 35S of cauliflower mosaic virus; wound-inducible promoter from tomato, leucine aminopeptidase ("LAP", Chao et al. (1999) Plant Physiol 120: 979-992); chemical inducible promoter from tobacco, pathogenesis related 1 (PR1) (induced by salicylic acid and BTH (benzothiadiazole-7-carbothioic acid S-methyl ester); tomato proteinase inhibitor II promoter (PIN2) or LAP promoter (both can be induced by methyl jasmonate); heat shock promoter (U.S. Pat. No. 5,187,267); tetracycline inducible promoter (U.S. Pat. No. 5,057,422); seed specific promoters, such as millet seed specific promoter pF128 (CN101063139B (Chinese Patent No. 200710099169.7)), seed storage protein specific promoters (for example, promoters of kidney bean globulin, napin, oleosin, and soybean beta conglycin (Beachy et al. (1985) EMBO J. 4: 3047-3053)). They can be used alone, or in combination with other plant promoters. All of the references cited herein are incorporated in their entireties. Suitable transcriptional terminators include, but are not limited to: *Agrobacterium* nopaline synthase terminator (NOS terminator), cauliflower mosaic virus CaMV 35S terminator, tml terminator, pea rbcS E9 terminator, and nopaline and octopine synthase terminator (see, for example, Odell et al. (1985) Nature 313:810; Rosenberg et al. (1987) Gene, 56:125; Guerineau et al. (1991) Mol. Gen. Genet, 262:141; Proudfoot (1991) Cell, 64:671; Sanfacon et al. Genes Dev., 5:141; Mogen et al. (1990) Plant Cell, 2:1261; Munroe et al. (1990) Gene, 91:151; Ballad et al. (1989) Nuclear Acids Res. 17:7891; Joshi et al. (1987) Nuclear Acid Res., 15:9627).

A recombinant vector containing the OsDREB 1C gene expression cassette can be constructed using the existing expression vector. The plant expression vector includes a binary *Agrobacterium* vector, and a vector that can be used for microprojectile bombardment in plant, etc. For example, pAHC25, pBin438, pCAMBIA1302, pCAMBIA2301, pCAMBIA1301, pCAMBIA1300, pBI121, pCAMBIA1391-Xa, PSN1301, or pCAMBIA1391-Xb (the CAMBIA company), etc. The plant expression vector can also contain the 3'-terminal untranslated region of a foreign gene, that is, an inclusion of poly(A) signals and any other DNA fragments involved in mRNA processing or gene expression. The poly(A) signals can lead poly(A) to be added to the 3'-terminal of mRNA precursor, and the untranslated regions transcribed at the 3'-terminal of a plasmid gene with crown gall induction by *Agrobacterium* (Ti) (such as nopaline synthase gene *NOS*) and a plant gene (such as soybean storage protein gene), for example, have similar functions. Enhancers, including translational enhancers or transcriptional enhancers, can also be used, when the gene of the invention is used to construct a plant expression vector. These enhancer regions can be ATG start codons, or start codons in adjacent regions, etc., but they must be the same as the reading frame of the coding sequence to ensure the correct translation of the whole sequence. The sources of the translational control signal and the start codons are extensive, and can be natural or synthetic. The translation initiation region can be derived from the transcriptional initiation region or structural genes. In order to facilitate the identification and screening of transgenic plant cells or plants, the plant expression vectors as used can be processed, such as by incorporating genes that can be expressed in plants and encodes enzymes or luminescent compounds that can bring about color changes (*GUS* gene, luciferase gene, etc.), antibiotic marker genes (such as the *nptII* gene that confers resistance to kanamycin and related antibiotics, the *bar* gene that confers resistance to a herbicide, phosphinothricin, the *hph* gene that confers resistance to an antibiotic, hygromycin, the *dhfr* gene that confers resistance to methotrexate, and the EPSPS gene that confers resistance to glyphosate), or anti-chemical reagent marker genes (such as a herbicide resistance gene), mannose-6-phosphate isomerase gene that enables mannose metabolism. Considering the safety of transgenic plants, the transformed plants can be screened directly under adverse circumstances, without incorporating any selective marker genes.

Among the uses above, the vector can be a plasmid, a cosmid, a phage, or a virus vector. The plasmid can be, in particular, pBWA(V)HS vector or psgR-Cas9-Os plasmid.

B3) the recombinant vector can be, in particular, pBWA(V)HS-*OsDREB1C.* The pBWA(V)HS-*OsDREB1C* is a recombinant vector obtained by inserting the OsDREB1C coding gene shown in SEQ ID NO: 2 in the Sequence Listing at BsaI (Eco31I) enzyme cleavage site of the pBWA(V)HS vector. The pBWA(V)HS-*OsDREB1C* can overexpress the protein encoded by *OsDREB1C* gene (i.e. the OsDREB1C protein shown in SEQ ID NO: 1), driven under the CaMV 35S promoter.

B8) the recombinant vector can be a recombinant vector prepared with crisper/cas9 system, which can reduce the content of OsDREB 1C. The recombinant vector can express sgRNA targeting the nucleic acid molecule described in B1). The target sequence of the sgRNA can be at positions 356-374 of SEQ ID NO: 2 in the Sequence Listing.

Among the uses above, the microorganism can be yeast, bacteria, algae, or fungi, wherein bacteria can be *Agrobacterium,* such as *Agrobacterium rhizogenes* EHA105.

Among the uses above, the transgenic plant cell line, transgenic plant tissue, and transgenic plant organ can include or exclude reproductive materials.

The invention further provides any of the methods as follows:
X1) a method for breeding a plant with increased yield, including expressing OsDREB1C in a recipient plant, or increasing the content or activity of OsDREB 1C in the recipient plant to obtain a target plant with increased yield;
X2) a method for breeding a plant with decreased yield, including lowering the content or activity of OsDREB1C in a recipient plant, or inhibiting the expression of OsDREB1C coding gene in the recipient plant to obtain a target plant with decreased yield compared to the recipient plant;
X3) a method for breeding a plant with enhanced photosynthesis and increased yield, including expressing OsDREB1C in a recipient plant, or increasing the content or activity of OsDREB1C in the recipient plant to obtain a target plant with enhanced photosynthesis and increased yield;
X4) a method for breeding a plant with enhanced photosynthesis, increased nitrogen uptake or transport capability, and increased yield, including expressing OsDREB1C in a recipient plant, or increasing the content or activity of OsDREB1C in the recipient plant to obtain a target plant with enhanced photosynthesis, increased nitrogen uptake or transport capability, and increased yield;
X5) a method for breeding a plant with enhanced photosynthesis, increased nitrogen content, and increased yield, including expressing OsDREB 1C in a recipient plant, or increasing the content or activity of OsDREB1C in the recipient plant to obtain a target plant with enhanced photosynthesis, increased nitrogen content, and increased yield;
X6) a method for breeding a plant with enhanced photosynthesis, enhanced nitrogen uptake or transport capability, earlier blooming stage, and increased yield, including expressing OsDREB1C in a recipient plant, or increasing the content or activity of OsDREB1C in the recipient plant to obtain a target plant with enhanced photosynthesis, enhanced nitrogen uptake or transport capability, earlier blooming stage, and increased yield;
X7) a method for breeding a plant with enhanced photosynthesis, increased nitrogen content, earlier blooming stage, and increased yield, including expressing OsDREB1C in a recipient plant, or increasing the content or activity of OsDREB1C in the recipient plant to obtain a target plant with enhanced photosynthesis, increased nitrogen content, earlier blooming stage, and increased yield.

Among the methods above, those described in X1) - X7) can be realized by introducing the coding gene of OsDREB1C into the recipient plant and expressing the coding gene.

Among the methods above, the coding gene can be the nucleic acid molecule described in B1).

Among the methods above, the coding gene of OsDREB1C can be modified as follows before being introduced into the recipient plant for better expression:
1) modifying and optimizing the coding gene of OsDREB1C according to actual requirement, to express the gene efficiently; for example, according to the codon bias of the recipient plant, the codons of amino acid sequence of the coding gene of OsDREB1C of the invention can be changed to conform to the codon bias of the plant while maintaining the amino acid sequence thereof; during optimization, it is preferable to maintain a certain content of GC in the optimized coding sequence, so as to achieve high-level expression of the gene introduced into the plant, in which the GC content can be 35%, more than 45%, more than 50%, or more than about 60%;
2) modifying the gene sequence adjacent to methionine, a start, to efficiently initiate translation; for example, by using effective sequences known in plants for the modifying;
3) linking to various plant expressing promoters to facilitate its expression in the plant; the promoter may include constitutive promoters, inducible promoters, temporal regulation promoters, developmental regulation promoters, chemical regulation promoters, tissue preferred promoters, and tissue specific promoters; the selection of promoters will vary with the requirement for expression time and space, and also depends on the target species; for example, a tissue- or organ- specific expression promoter depends on a desired phase of development of a recipient; although it has been shown that many promoters from a dicotyledon plant may function in a monocotyledon plant, and vice versa, dicotyledon promoters are ideally selected for expression in a dicotyledon plant, and monocotyledon promoters for expression in a monocotyledon plant;
4) linking to a suitable transcriptional terminator, which can also increase the expression efficiency of the gene of the invention; for example, tml derived from CaMV, and E9 derived from rbcS; any of those available terminators known to function in a plant can be linked to the gene of the invention;
5) introducing enhancer sequence(s), such as intron sequences (e.g. derived from Adhl and bronzel) and viral leading sequences (e.g. derived from TMV, MCMV, and AMV).

The coding gene of OsDREB1C can be introduced into a recipient plant using a recombinant expression vector containing the coding gene of OsDREB1C. The recombinant expression vector can be, in particular, the pBWA(V)HS-*OsDREB1C.*

The recombinant expression vector can be introduced into plant cells by using routine biotechnological approaches such as Ti plasmid, a plant virus vector, direct DNA transformation, microinjection, electroporation, etc. (Weissbach, 1998, Method for Plant Molecular Biology VIII, Academy Press, New York, pp.411-463; Geiserson and Corey, 1998, Plant Molecular Biology (2nd Edition).).

The target plant is construed to include not only first generation of plant with the protein OsDREB1C or coding gene thereof modified, but also its progeny (filial generation). For the target plant, the gene can be reproduced in this species, or the gene can be transferred into other cultivars of the same species, and especially, into commercial cultivars, using conventional breeding techniques. The target plant includes seeds, callus, intact plants, and cells.

The invention also provides a product having any of the following functions D1) - D6), the product comprises OsDREB 1C or the biological material:
D1) regulation of yield of a plant;
D2) regulation of photosynthesis and yield of a plant;
D3) regulation of nitrogen uptake or transport in, photosynthesis, and yield of a plant;
D4) regulation of nitrogen content in, photosynthesis, and yield of a plant;
D5) regulation of nitrogen uptake or transport in, photosynthesis, blooming stage of, and yield of a plant;
D6) regulation of nitrogen content in, photosynthesis, blooming stage, and yield of a plant.

In the above, the plant can be M1) or M2) or M3):
M1) a monocotyledon, or dicotyledon plant;
M2) a gramineous plant, a cruciferous plant, or a leguminous plant;
M3) rice (*Oryza sativa* L.), wheat (*Triticum aestivum),* maize (*Zea mays),* Arabidopsis (*Arabidopsis thaliana), Brassica napus* L., or soybean (*Glycine max).*

In the above, the photosynthesis can be reflected in photosynthetic rate, net photosynthetic rate, heat dissipation NPQ, maximum carboxylation efficiency, and/or maximum electron transfer rate;
the transport can be reflected in a transport from roots to above-ground parts, or to grains;
the nitrogen content can be nitrogen content in a plant or in an organ;
the blooming stage can be reflected in heading stage;
the yield can be reflected in grain yield, above-ground yield, and/or harvest index.

The organ can be roots, stems, leaves, and/or grains of the plant.

The harvest index refers to the ratio of economic yield (grains, fruits, etc.) to biological yield in plant harvest.

The regulation can be enhanced or inhibited, promoted or inhibited, or, increased or decreased.

Another technical problem to be solved by the invention is how to detect yield traits of rice.

In order to solve the above technical problem, the invention first provides a method for detecting yield traits of rice, comprising: detecting haplotypes of rice to be tested, the haplotypes of the rice being Hap.1, Hap.2 and Hap.3, the Hap. 1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the yield traits of the rice to be tested are identified according to the following steps: homozygous rice to be tested having Hap. 2 as haplotype has lower yield, homozygous rice to be tested having Hap. 3 as haplotype has higher yield, and there is no difference in yield between homozygous rice to be tested having Hap. 1 as haplotype and both the homozygous rice to be tested having haplotype of Hap. 2 and the homozygous rice to be tested having haplotype of Hap. 3.

The invention also provides another method for detecting yield traits of rice, comprising: detecting haplotypes of rice to be tested, with the haplotypes of the rice being Hap.1, Hap.2 and Hap.3, the Hap. 1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the yield traits of the rice to be tested are identified according to the following steps: homozygous rice to be tested having Hap. 2 as haplotype has lower grain yield per plant than that of homozygous rice to be tested having Hap. 3 as haplotype, and there is no difference in grain yield per plant between rice to be tested having Hap. 1 as haplotype and both homozygous ice to be tested having Hap. 2 as haplotype of and the homozygous rice to be tested having Hap. 3 as haplotype.

The invention also provides a method for breeding rice, comprising: detecting nucleotides in rice genome DNA at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/), and selecting the rice having nucleotides, in sequence, A, A, G, C, A, G, G, C, T, and G, or G, G, A, G, T, A, A, T, G, and T at positions 1433007, 1433155, 1433229, 1433365, 1433528, 1433919, 1434216, 1434258, 1434486, and 1434806 as parent plants for breeding.

The invention also provides use of a material for detecting rice haplotypes in detecting yield traits of rice, with the haplotypes of the rice being Hap.1, Hap.2 and Hap.3, the Hap. 1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/).

The invention also provides use of a material for detecting rice haplotypes in the preparation of a product for detecting yield traits of rice, with the haplotypes of the rice being Hap.1, Hap.2 and Hap.3, the Hap. 1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0) (updated on February 6, 2013, website: https://rapdb.dna.affrc.go.jp/).

In the invention, the material for detecting rice haplotypes can be used to detect yield traits of the rice. The material for detecting rice haplotypes can be a primer and/or a probe capable of detecting the Hap.1, the Hap.2, and the Hap. 3, and can also be a kit and/or an instrument required for sequencing (such as First-Generation Sequencing or High-Throughput Sequencing).

### DESCRIPTION OF THE DRAWINGS

Figure 1 shows the results of sequence alignment.
Figure 2 shows the detection of the relative expression level of *OsDREB1C* gene in transgenic rice, and the sequence detection of a target region of a rice material with the gene knocked out. (A) The relative expression level of *OsDREB1C* gene; (B) sites for gene editing in rice with *Osdreb1c* gene knocked out.
Figure 3 shows the photosynthetic parameters for wild-type and transgenic rice plants. A - diurnal variation of photosynthesis; B - diurnal variation of NPQ; C - light response curves; D - CO₂ response curves; E - maximum CO₂ carboxylation efficiency; F - maximum electron transfer rate.
Figure 4 shows the detection of nitrogen uptake and utilization in wild-type and transgenic rice plants. A - ¹⁵N content of above-ground parts; B - ¹⁵N content in roots; C - ¹⁵N uptake efficiency; D - ¹⁵N transport efficiency from roots to above-ground parts; E - nitrogen contents in different tissues of rice; F - distribution ratio of nitrogen in different tissues of rice. Grains, straws and leaves are arranged from top to bottom in sequence within the column diagrams of E and F.
Figure 5 shows the detection of BAR gene in transgenic wheat (A), and the relative expression level of *OsDREB1C* gene in transgenic *Arabidopsis thaliana* (B).
Figure 6 shows the phenotypes of wild-type and transgenic wheat. (A) The phenotypes of wild-type (Fielder) and transgenic wheat. (B-D) Heading stage (B), photosynthetic rate (C), grain number (D), 1000-grain weight (E), and grain yield per plant (F) of wild-type and transgenic lines.
Figure 7 shows the phenotypic characteristics of wild-type and transgenic *Arabidopsis thaliana.* (A) The phenotypes of wild-type Col-0 and transgenic lines of *Arabidopsis thaliana.* (B-D) Bolting stage (B), number of rosette leaves (C), and above-ground fresh weight (D) of the wild-type and transgenic lines of *Arabidopsis thaliana.*
   *P < 0.05, **P < 0.01.
Figure 8 is sequence analysis for three haplotypes.
Figure 9 is analysis of yields of three haplotypes. The vertical coordinate represents grain yield per plant, with Hap1, Hap2 and Hap3 representing Hap.1, Hap.2 and Hap.3. Different letters indicate significant differences between haplotypes (P<0.05, Duncan multiple range test).

### BEST MODES OF CARRYING OUT THE EMBODIMENTS

The invention is further described in detail below in combination with specific embodiments. The examples are provided only for the purpose of illustrating the invention, without limiting the scope of the invention. The examples provided below can be used as a guide for an ordinary skilled in the art for further improvement, and do not constitute a limitation to the invention in any way.

The experimental methods in the following examples, unless otherwise specifically stated, are routine methods, which are carried out in accordance with the techniques or conditions described in the literatures in the art, or in accordance with the product's instructions. The materials, reagents, instruments used in the following examples are all commercially available, unless otherwise specifically indicated. The quantitative assays in the following examples are all repeated in triplicate, the results of which are averaged. In the following examples, unless otherwise specifically specified, the first position of each nucleotide sequence in the Sequence Listing is the 5'-terminal nucleotide of the respective DNA/RNA, and the last position is the 3'-terminal nucleotide of the respective DNA/RNA.

The biological material, pBWA(V)HS vector (Zhao et al., DEP1 is involved in regulating the carbon-nitrogen metabolic balance to affect grain yield and quality in rice (Oriza sativa L.), PLOS ONE, March 11, 2019, https://doi.org/10.1371/journal.pone.0213504) in the following examples is available to the general public from the applicant, the biological material is merely intended to be used for replicating the relevant experiments in the invention and by no means for other purposes.

The biological material, psgR-Cas9-Os containing plasmid (Xuejiao Hu, Jia Yang, Can Cheng, Jihua Zhou, Fu'an Niu, Xinqi Wang, Meiliang Zhang, Liming Cao, Huangwei Chu. Targeted Editing of Rice SD1 Gene Using CRISPR/Cas9 System. Chinese Journal of Rice Science, 2018, 32(3): 219-225; Mao Y, Zhang H, Xu N, Zhang B, Gou F, Zhu J K. Application of the CRISPR-Cas system for efficient genome engineering in plants. Mol Plant, 2013, 6(6): 2008-2011.) in the following examples is available to the general public from the applicant, the biological material is merely intended to be used for replicating the relevant experiments in the invention and by no means for other purposes.

### Example 1: OsDREB1C can function to enhance photosynthetic efficiency, facilitate nitrogen uptake, promote early heading, and increase yield of rice.

This example provides a protein from rice Nipponbare, which can function to enhance the photosynthetic efficiency, facilitate nitrogen uptake, promote early heading, and increase yield of rice, referred to as OsDREB1C, sequence of which is shown as SEQ ID NO: 1 in the Sequence Listing. In Nipponbare, the coding gene sequence of OsDREB1C is SEQ ID NO: 2, and the genome sequence is at positions 3001-4131 of SEQ ID NO: 3. In SEQ ID NO: 3, positions 1-3000 are the promoter of OsDREB 1C gene in the genomic DNA of Nipponbare.

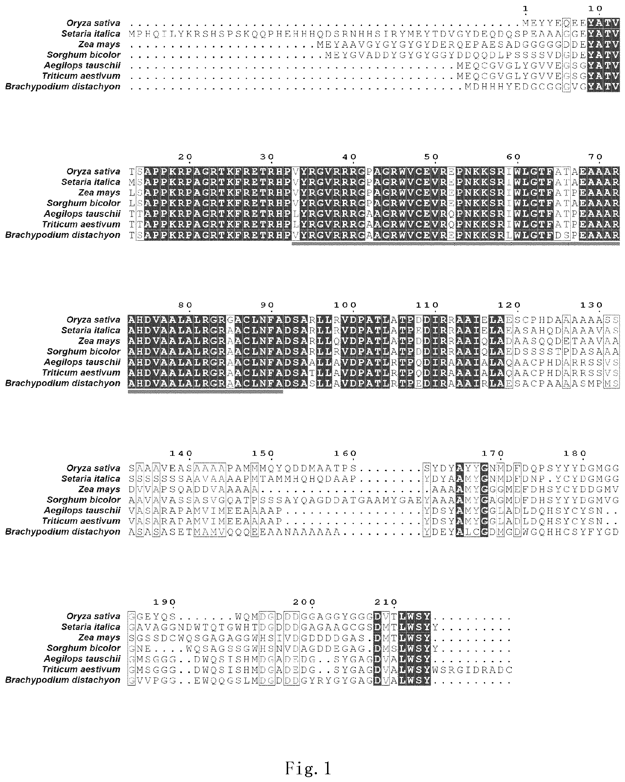

The sequence alignment between rice OsDREB1C and homologous proteins in other plants reveals that the rice OsDREB1C has 73.52%, 64.06%, 66.52%, 66.05%, 66.05% and 65.88% identity with that from millet *(Setaria italic),* maize (*Zea mays),* sorghum (*Sorghum bicolor), Aegilops tauschii,* wheat *(Triticum aestivum)* and *Brachypodium distachyon,* respectively (Fig. 1).

### 1. Construction of a recombinant vector

**Construction of an overexpression vector:** PCR products containing full-length CDS of *OsDREB1C* gene were obtained by amplifying from cDNA of rice Nipponbare by PCR. The resulting PCR products were cleaved with BsaI (Eco31I) alone, and the resulting enzymatically cleaved products were linked to the vector backbone of the PBWA(V)HS vector obtained by being cleaved with BsaI (Eco31I) alone, to give a recombinant vector with correct sequence, designated as PBWA(V)HS-*OsDREB1C*. pBWA(V)HS-*OsDREB1C* is a recombinant vector obtained by inserting the OsDREB1C coding gene shown in SEQ ID NO: 2 in the Sequence Listing at the BsaI (Eco31I) enzyme cleavage of the PBWA(V)HS vector. pBWA(V)HS-*OsDREB1C* can be driven under CaMV 35S promoter to express the protein encoded by OsDREB1C gene (i.e. the protein OsDREB1C shown in SEQ ID NO: 1).

The primer sequences used are as follows:
*OsDREB1C*-F: 5'-CAGTGGTCTCACAACATGGAGTACTACGAGCAGGAGGAGT-3' (SEQ ID NO: 4 in the Sequence Listing);
*OsDREB1C*-R: 5'-CAGTGGTCTCATACATCAGTAGCTCCAGAGTGTGACGTCG-3' (SEQ ID NO: 5 in the Sequence Listing).

**Construction of a gene knockout vector:** sgRNA and primers were designed on an online design website (http://skl.scau.edu.cn/) to have a target sequence determined as 5'-AGTCATGCCCGCACGACGC-3' (positions 356-374 in SEQ ID NO: 2). *OsDREB1C*-sgRNA-F and *OsDREB1C*-sgRNA-R were annealed, the resulting products were cleaved by BsaI, and the resulting enzymatically cleaved products were linked to the vector backbone of the psgR-Cas9-Os containing plasmid obtained by being cleaved with BsaI, to give a recombinant vector with correct sequence, an *OsDREB1C* gene knockout vector, designated as OsU3-sgRNA-OsUBI-Cas9-*OsDREB1C*. In this recombinant vector, OsU3 promoter drove sgRNA, and OsUBI promoter drove Cas9.

In the above, the primer sequences used are as follows:
*OsDREB1C*-sgRNA-F: 5'-TGTGTGGCGTCGTGCGGGCATGACT-3' (SEQ ID NO: 6 in the Sequence Listing);
*OsDREB1C*-sgRNA-R: 5'-AAACAGTCATGCCCGCACGACGCCA-3' (SEQ ID NO: 7 in the Sequence Listing).

### 2. Construction of a transgenic plant

After disinfecting the mature seeds of Nipponbare, a cultivar of Oryza sativa subsp. Keng of rice, they were induced to provide embryogenic callus. PBWA(V)HS-*OsDREB1C* and OsU3-sgRNA-OsUBI-Cas9-*OsDREB1C* obtained in step 1 were introduced into *Agrobacterium* EHA105, respectively. The callus was transfected and co-cultured with *Agrobacterium* using a genetic transformation method for rice, mediated by *Agrobacterium,* to provide a transgenic plant upon resistance screening. The screened transgenic rice derived from PBWA(V)HS-*OsDREB1C* is an *OsDREB1C* transgenic rice, and the transgenic rice derived from OsU3-sgRNA-OsUBI-Cas9-*OsDREB1C* is an *OsDREBIC* gene knockout rice material.

The relative expression levels of *OsDREB1C* gene at RNA level in the *OsDREB1C* transgenic rice and the *OsDREB1C* gene knockout rice were detected by qRT-PCR assay using Nipponbare (WT), a cultivar of *Oryza sativa* subsp. *japonica* (Geng) of rice as a control. The primers used were: 5'-CATGATGATGCAGTACCAGGA-3' (SEQ ID NO: 8 in the Sequence Listing), 5'-GATCATCAGTAGCTCCAGAGTG-3' (SEQ ID NO: 9 in the Sequence Listing); the internal reference gene is rice Ubiquitin, and the primers for the internal reference gene are: 5'-AAGAAGCTGAAGCATCCAGC-3' (SEQ ID NO: 10 in the Sequence Listing), 5'-CCAGGACAAGATGATCTGCC-3' (SEQ ID NO: 11 in the Sequence Listing).

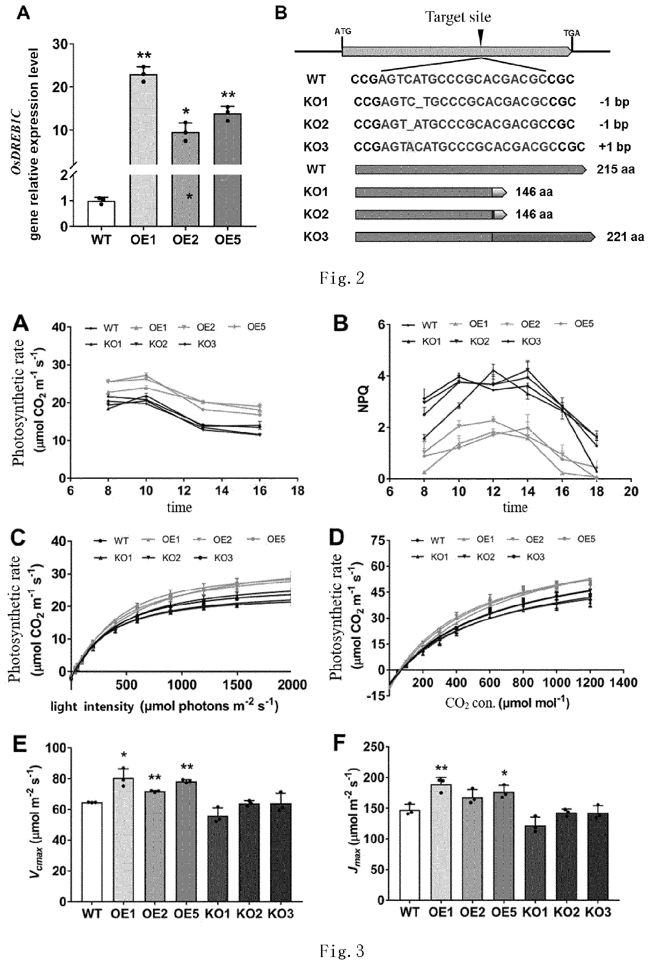

The results showed that the relative expression level of *OsDREB1C* gene in each of three lines (OE1, OE2 and OE5) of the *OsDREB1C* transgenic rice was significantly higher than that of the wild type (WT), and the three lines were rice materials that overexpress *OsDREB1C* (A of Fig. 2). The presence of base deletion or insertion in the sequence of *OsDREB1C* gene in three lines (KO1, KO2 and KO3) of the *OsDREB1C* gene knockout rice material caused frame shift mutation, thereby resulting in loss of normal function of the *OsDREB1C* protein (B of Fig. 2).

PCR amplification and sequencing were carried out on the three lines (KO1, KO2 and KO3) of the *OsDREB1C* gene knockout rice material using primers that can amplify the target sequence, as well as the upstream and downstream sequences thereof. The results showed the changes of the target sequence in the three lines, as shown in B of Fig. 2: deletion of one nucleotide occurred in both KO1 and KO2, insertion of one nucleotide occurred in KO3, and frame shift mutations occurred in each of the target genes of the three lines.

### 3. Enhancement of photosynthetic efficiency in OsDREB1C transgenic rice

The photosynthesis indexes of field-grown rice were measured. The rice to be tested: wild-type rice Nipponbare (WT), *OsDREB1C* overexpressing rice (OE1/OE2/OE5), and *OsDREB1C* gene knockout rice (KO1/ KO2/ KO3).

The flag leaves of the rice to be tested at heading stage were measured using LICOR-6400XT Portable Photosynthesis System (LI-COR, USA) for diurnal variation of photosynthesis, light response curves, and CO₂ response curves, respectively, which reflected the photosynthetic capacity of a plant. The diurnal variation of photosynthesis was measured once every 2-4 hours from 8:00 to 16:00 in sunny and cloudless weather. The photosynthetic rate was measured using LICOR-6400XT Portable Photosynthesis System, and NPQ (non-photochemical quenching) was measured using FluorPen FP100 (PSI, Czech), where the leaves needed to be in dark adaptation for 15-20 minutes prior to NPQ measurement. In the measurement of light response curves, the CO₂ was set at a concentration of 400 µmοl mol⁻¹, and the light intensity (PPFD) at 0 to 2000 µmοl m⁻² s⁻¹. In the measurement of CO₂ response curves, PPFD was set at 1200 µmοl m⁻² s⁻¹, and CO₂ concentration decreased from 400 to 50 µmοl mol⁻¹, then increased from 400 µmοl mol⁻¹ to 1200 µmοl mol⁻¹ again. Both light response curves and CO₂ response curves were fitted by Farquhar-von Caemmerer-Berry (FvCB) model, and maximum carboxylation efficiency (*V_{cmax}*) and maximum electron transfer rate (*Jₘₐₓ*) were calculated from the CO₂ response curves.

The results showed that the photosynthetic efficiency (i.e. photosynthesis efficiency) of *OsDREB1C* overexpressing rice was each significantly higher than that of the wild type in the daytime, with a peak in the difference at noon when the light intensity was highest, from which a significant level was observed - an increase by 29.5-43.3% compared with the wild type. At the same time, NPQ which involves consumption of heat dissipation of excess absorbed energy was significantly lower than that in the wild type (A, B in Fig. 3, and Tables 1 and 2), and the differences each reached a significant level, suggesting that more light energy was involved in photosynthesis. Further, the measurements of light response curves and CO₂ response curves showed that when the light intensity was less than 200 µmοl m⁻² s⁻¹, there is no significant difference in net photosynthetic rate between the *OsDREB1C* overexpressing rice and the wild-type rice, while a gradually growing difference was observed under high light intensity - an increase in the photosynthetic rate, under the light intensity of 2000 µmοl m⁻² s⁻¹, of the *OsDREB1C* overexpressing rice by 18.4-27.6% compared with the wild type (C in Fig. 3, and Table 3) - the difference has reached a significant level. The measurement of CO₂ response curves was similar to that of light response curves, the photosynthetic rate of *OsDREB1C* overexpressing rice was significantly higher than that of the wild type when CO₂ concentration was greater than 400 µmοl mol⁻¹ (D in Fig. 3, and Table 4). Further calculation showed that both the maximum carboxylation efficiency (*V_{cmax}*) and maximum electron transfer rate (*Jₘₐₓ*) were significantly higher than that of the wild type (E, F in Fig. 3, and Table 5). Still, photosynthesis associated parameters for the *OsDREB1C* gene knockout rice were lower than, or close to those of the wild type. The above results indicate that, overexpression of *OsDREB1C* gene in rice can significantly enhance both light use efficiency and CO₂ assimilation capability.

**Table 1. Measurements of Photosynthetic Efficiency (µmοl CO₂ m⁻² s⁻¹**

| Time | WT | OE1 | OE2 | OE5 | KO1 | KO2 | KO3 |
|---|---|---|---|---|---|---|---|
| 8:00 | 21.59±1.42 | 22.78±1.08 | 25.51±1.76^{∗∗} | 25.53±0.80^{∗∗} | 18.44±0.80 | 19.35±1.49^{∗} | 20.41±0.70 |
| 10:00 | 20.78±2.53 | 23.94±1.41^{∗} | 26.26±0.49^{∗∗} | 27.18±1.69^{∗∗} | 21.88±1.42 | 20.49±2.43 | 19.74±1.83 |
| 13:00 | 14.07±0.95 | 20.16±0.80^{∗∗} | 20.12±0.76^{∗∗} | 18.22±0.63^{∗∗} | 13.77±1.19 | 12.81±1.31 | 13.41±2.29 |
| 16:00 | 13.44±1.59 | 18.08±1.79^{∗∗} | 19.03±2.09^{∗∗} | 16.78±1.57^{∗∗} | 14.00±2.61^{∗} | 11.44±0.79^{∗} | 11.55±0.91^{∗} |

In Table 1, compared with WT at the same place in the same year, * indicates that the difference has reached a significant level (p < 0.05); ** indicates that the difference has reached an extremely significant level (p < 0.01).

**Table 2. Measurements of NPQ**

| Time | WT | OE1 | OE2 | OE5 | KO1 | KO2 | KO3 |
|---|---|---|---|---|---|---|---|
| 8:00 | 3.12±0.76 | 0.25±0.13^{∗∗} | 1.03±0.74^{∗∗} | 0.89±0.58^{∗∗} | 1.60±0.25^{∗} | 2.96±0.32 | 2.50±0.57 |
| 10:00 | 3.97±0.28 | 1.36±0.64^{∗∗} | 2.05±0.43^{∗∗} | 1.21±0.74^{∗∗} | 2.85±0.21^{∗∗} | 3.78±0.53 | 3.75±0.54 |
| 12:00 | 3.45±0.07 | 1.83±0.23^{∗∗} | 2.26±0.33^{∗∗} | 1.72±0.25^{∗∗} | 4.23±0.47^{∗} | 3.67±0.44 | 3.66±0.80 |
| 14:00 | 3.62±0.20 | 1.58±0.80^{∗} | 1.65±1.13^{∗} | 1.98±1.05^{∗} | 3.32±0.45 | 4.23±0.72 | 3.95±1.19 |
| 16:00 | 2.70±0.24 | 0.23±0.12^{∗∗} | 0.95±0.73^{∗} | 0.76±0.65^{∗∗} | 2.65±0.66 | 2.82±0.67 | 2.82±0.31 |
| 18:00 | 0.28±0.33 | 0.06±0.07 | 0.01±0.02 | 0.45±0.51 | 1.63±0.24^{∗∗} | 1.62±0.49 | 1.28±0.43^{∗} |

In Table 2, compared with WT at the same place in the same year, * indicates that the difference has reached a significant level (p < 0.05); ** indicates that the difference has reached an extremely significant level (p < 0.01).

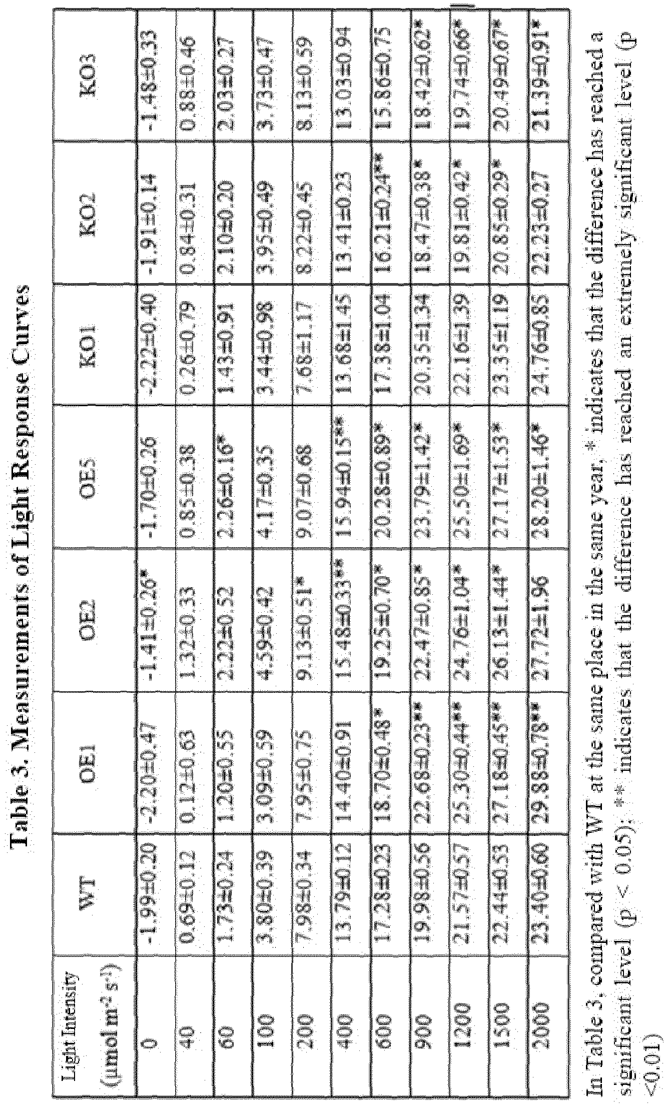

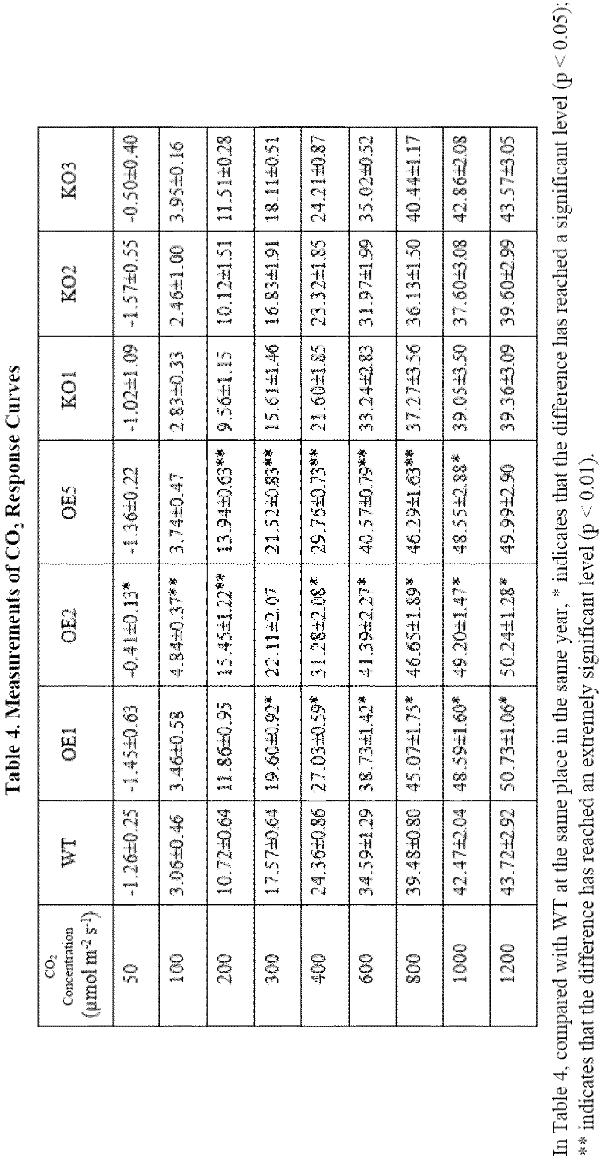

**Table 5. Results of Maximum Carboxylation Efficiency (V_{cmax}) and Maximum Electron Transfer Rate (Jₘₐₓ)**

| Rice | Maximum Carboxylation Efficiency (*V_{cmax}*) (µmol m⁻² s⁻¹) | Maximum Electron Transfer Rate (*Jₘₐₓ*) (µmol m⁻² s⁻¹) |
|---|---|---|
| WT | 64.53±0.13 | 147.28±9.19 |
| OE1 | 80.43±6.02^{∗} | 188.51±11.70^{∗∗} |
| OE2 | 71.73±0.55^{∗∗} | 168.36±12.19 |
| OE5 | 78.29±1.01^{∗∗} | 176.31±10.78^{∗} |
| KO1 | 55.84±5.16 | 122.29±13.20 |
| KO2 | 63.90±1.66 | 142.99±5.71 |
| KO3 | 63.95±6.51 | 142.65±11.51 |

In Table 5, compared with WT at the same place in the same year, * indicates that the difference has reached a significant level (p < 0.05); ** indicates that the difference has reached an extremely significant level (p < 0.01).

### 4. Increase in nitrogen use efficiency in OsDREB1C transgenic rice

Rice was measured for nitrogen use efficiency. The rice to be tested: wild-type rice Nipponbare (WT), *OsDREB1C* overexpressing rice (OE1/OE2/OE5), and *OsDREB1C* gene knockout rice (KO1/ KO2/ KO3).

Rice seedlings to be tested, which were hydroponically cultured in a greenhouse for 3 weeks, were placed in Kimura B solution without nitrogen (free of (NH₄)₂SO₄ and KNO₃) in advance for nitrogen starvation treatment for 3 days. After the nitrogen starvation treatment, the roots of the seedlings were completely immersed in 0.1 mM CaSO₄ solution (deionized water as the solvent) for 1 minute before the residual water was sucked up, and then they were cultured by placing the roots in a nutrient solution containing 0.5 mM K¹⁵NO₃. 3 hours later, the roots of the seedlings were again immersed in 0.1 mM CaSO₄ solution for 1 minute, after which the residual water was sucked up, followed by collecting the respective above-ground parts and roots for dryness at 70°C for 3 days to constant weights, and the dry weights of the samples were recorded. After the samples were ground and crushed, ¹⁵N contents in the respective above-ground parts and roots were measured using IsoPrime 100 stable isotope ratio mass spectrometer (Elementar, Germany), and nitrogen uptake efficiency and nitrogen transport efficiency were calculated. Nitrogen uptake efficiency = (¹⁵N contents in above-ground parts + ¹⁵N contents in roots) / dry weights of roots / 3; Nitrogen transport efficiency = ¹⁵N contents in above-ground parts / ¹⁵N contents in roots.

In addition, mature rice plants to be tested grown in Beijing field were harvested, and leaves, straws and grains of a single plant were taken for kill-green at 105°C for 30 minutes and dried at 70°C for 3 days. After the samples were ground and crushed, nitrogen contents were separately measured using IsoPrime 100 stable isotope ratio mass spectrometer (Elementar, Germany), and distribution ratios of nitrogen in different parts were calculated.

The nutrient solutions used were as follows:
Nutrient solution: Kimura B nutrient solution (0.5 mM (NH₄)₂SO₄, 1 mM KNO₃, 0.54 mM MgSO₄·7H₂O, 0.3 mM CaCl₂, 0.18 mM KH₂PO₄, 0.09 mM K₂SO₄, 16 µM Na₂SiO·9H₂O, 9.14 µM MnCl₂·4H₂O, 46.2 µM Na₂MoO₄·2H₂O, 0.76 µM ZnSO₄·7H₂O, 0.32 µM CuSO₄·5H₂O, 40 µM Fe(II)-EDTA, pH= 5.8).

Nutrient solution containing 0.5 mM K¹⁵NO₃: Kimura nutrient solution without nitrogen which has been incorporated a dilution of 0.5 M K¹⁵NO₃ stock solution by 1000-fold (0.54 mM MgSO₄·7H₂O, 0.3 mM CaCl₂, 0.18 mM KH₂PO₄, 0.09 mM K₂SO₄, 16 µM Na₂SiO₃·9H₂O, 9.14 µM MnCl₂·4H₂O, 46.2 µM Na₂MoO₄·2H₂O, 0.76 µM ZnSO₄·7H₂O, 0.32 µM CuSO₄·5H₂O, 40 µM Fe(II)-EDTA, pH= 5.8).

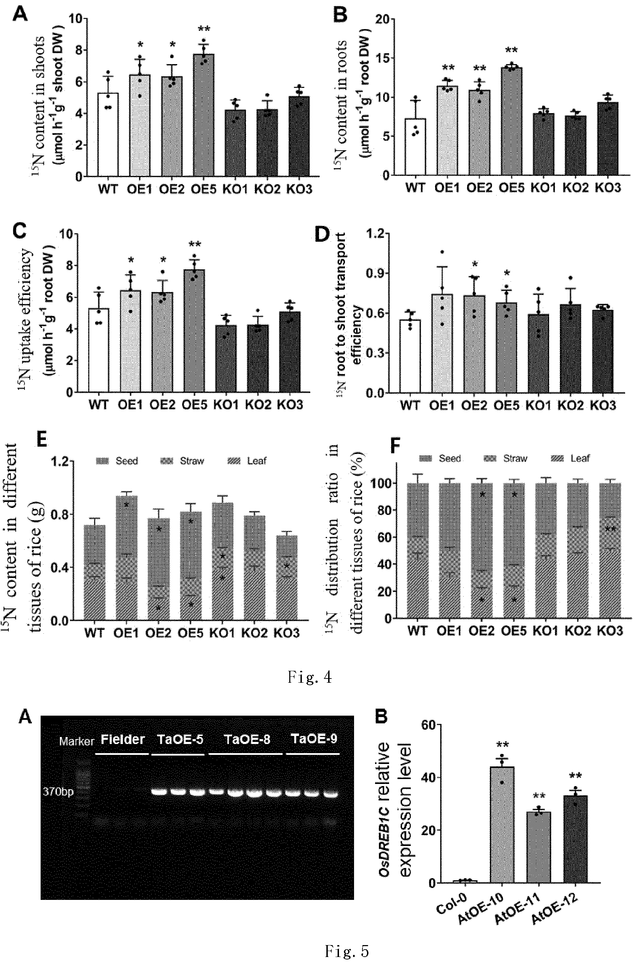

The results showed that after 3 hours of ¹⁵N treatment, ¹⁵N contents in above-ground parts and roots of the *OsDREB1C* overexpressing rice were significantly higher than that of the wild type (A, B of Fig. 4, and Table 6), which was primarily attributed to the significant rise over the wild type in nitrogen uptake efficiency in roots (C of Fig. 4, Table 6), and in transport efficiency of nitrogen from roots to above-ground parts (D of Fig. 4, Table 6); while the difference between the *OsDREB1C* gene knockout rice and the wild type was not obvious: its indexes were approximate to that of the wild type, except for a lower ¹⁵N uptake efficiency than that of the wild type. Further, analysis of nitrogen distribution in different tissues of a field-grown rice plant suggested a general increase in nitrogen content in a whole plant of *OsDREB1C* overexpressing rice than in the wild type (E of Fig. 4, Table 7), with 50.3-66.4% of the nitrogen distributed to grains, much higher than 41.1% for the wild type, and 26-38.6% for the *OsDREB1C* knockout rice (F of Fig. 4, table 7). At the same time, the nitrogen allocated to leaves and straws decreased correspondingly, at 21.1-29.7% and 12.5-19.9%, respectively; while the ratios in the wild type were 42.66% and 16.28%, respectively, with 43.2-49.3% and 18.2-24.7% for the *OsDREB1C* knockout rice (F of Fig. 4). From the above results, overexpression of *OsDREB1C* gene in rice can significantly increase the efficiency of nitrogen uptake and transport in a plant, and allocate more nitrogen to grains.

**Table 6. Measurements of Indexes of Rice Cultivated in Greenhouse**

| Rice | Greenhouse | | | |
|---|---|---|---|---|
| | ¹⁵N Contents in Above-Ground Parts (µmol ¹⁵N/hour/g DW) | ¹⁵N Contents in Roots (µmol ¹⁵N/hour/g DW) | ¹⁵N Uptake Efficiency | Efficiency of ¹⁵N Transport from Roots to Above-Ground Parts |
| WT | 1.45±0.18 | 7.30±2.30 | 5.00±0.83 | 0.55±0.06 |
| OE1 | 2.17±0.10^{∗∗} | 11.50±0.69^{∗} | 6.46±0.94^{∗} | 0.74±0.20 |
| OE2 | 2.20±0.30^{∗∗} | 10.95±1.05^{∗} | 6.33±0.74^{∗} | 0.73±0.14^{∗} |
| OE5 | 2.43±0.15^{∗∗} | 13.81±0.34^{∗∗} | 7.75±0.61^{∗∗} | 0.68±0.09^{∗} |
| KO1 | 1.45±0.38 | 7.96±0.58 | 4.24±0.62 | 0.59±0.15 |
| KO2 | 1.66±0.17 | 7.67±0.50 | 4.27±0.53 | 0.67±0.12 |
| KO3 | 1.53±0.22 | 9.39±0.90 | 5.10±0.55 | 0.63±0.04 |

In Table 6, compared with WT at the same place in the same year, * indicates that the difference has reached a significant level (p < 0.05); ** indicates that the difference has reached an extremely significant level (p < 0.01).

**Table 7. Measurements of Indexes of Field-Cultivated Rice**

| Rice | Nitrogen Contents (g/plant) | | | Nitrogen Distribution Ratio (%) | | |
|---|---|---|---|---|---|---|
| | Grains | Straws | Leaves | Grains | Straws | Leaves |
| WT | 0.30±0.05 | 0.12±0.01 | 0.3±0.03 | 41.06±6.42 | 16.28±1.61 | 42.66±5.51 |
| OE1 | 0.47±0.03^{∗} | 0.19±0.03 | 0.28±0.04 | 50.39±3.12 | 19.92±2.86 | 29.69±3.93 |
| OE2 | 0.52±0.07^{∗} | 0.09±0.01 | 0.16±0.01^{∗} | 66.40±3.19^{∗} | 12.51±1.60 | 21.09±1.63^{∗} |
| OE5 | 0.51±0.06^{∗} | 0.13±0.01 | 0.18±0.01^{∗} | 61.92±2.61^{∗} | 16.15±1.53 | 21.94±2.01^{∗} |
| KO1 | 0.35±0.05 | 0.16±0.01^{∗∗} | 0.38±0.02^{∗} | 38.59±3.88 | 18.19±1.04 | 43.23±2.88 |
| KO2 | 0.27±0.03 | 0.15±0.02 | 0.37±0.04 | 33.85±2.77 | 19.25±1.41 | 46.89±1.48 |
| KO3 | 0.17±0.03 | 0.16±0.01^{∗∗} | 0.31±0.02 | 26.01±2.61 | 24.69±0.87^{∗∗} | 49.30±2.20 |

In Table 7, compared with WT at the same place in the same year, * indicates that the difference has reached a significant level (p < 0.05); ** indicates that the difference has reached an extremely significant level (p < 0.01).

### 5. Early heading stage of OsDREB1C transgenic rice

Rice was examined for their heading stages. The rice to be tested: wild-type rice Nipponbare (WT), *OsDREB1C* overexpressing rice (OE1/OE2/OE5), and *OsDREB1C* gene knockout rice (KO1/ KO2/ KO3).

The heading stage of each rice to be tested was counted under field growing conditions in Beijing. The statistical method was as follows: each type of the rice to be tested was grown in triplicate plots in a random arrangement; the moment when the spikes of 50% of the plants in the plot were unsheathed by 1/2 of the flag leaf sheaths was recorded as heading; and the heading stage was the days from sowing to heading.

The results showed (Table 8) that the heading stage of the *OsDREB1C* overexpressing rice was substantially advanced than that of the wild type, 13-17 days earlier in 2018 and 17-19 days earlier in 2019, while the heading stage of the *OsDREB1C* gene knockout rice was 6-8 days and 3-5 days later than that of the wild type, respectively; grain filling and maturity stage of rice was advanced or postponed, correspondingly. This indicated that *OsDREB1C* and coding gene thereof can regulate the heading stage of rice.

**Table 8. Heading Stages of Wild-Type and Transgenic Rice under Field Conditions in Beijing (Days)**

| Rice | **Year of 2018** | **Year of 2019** |
|---|---|---|
| WT | 117±1 | 120.6±0.5 |
| OE1 | 99.3±0.5 ^{∗∗} | 103±1 ^{∗∗} |
| OE2 | 103.3±0.5 ^{∗∗} | 101.3±0.5 ^{∗∗} |
| OE5 | 101.6±0.5 ^{∗∗} | 103±1 ^{∗∗} |
| KO1 | 123.7±0.58 ^{∗∗} | 124.7±0.58 ^{∗∗} |
| KO2 | 123.3±0.58 ^{∗∗} | 124±0 ^{∗∗} |
| KO3 | 124.7±0.58 ^{∗∗} | 123.7±0.58 ^{∗∗} |

In Table 8, compared with WT at the same place in the same year, ** indicates that the difference has reached an extremely significant level (p < 0.01).

### 6. A substantial rise in yield, quality, and harvest index of field-grown transgenic rice

The above-ground dry weight and grain yield of rice were measured. The rice to be tested: wild-type rice Nipponbare (WT), *OsDREB1C* overexpressing rice (OE1/OE2/OE5), and *OsDREB1C* gene knockout rice (KO1/ KO2/ KO3).

In the field experiments carried out in Beijing and Hainan, separately, each type of the rice to be tested was grown in triplicate plots in a random arrangement. After rice grains were mature, grain yield per plant, grain yield per plot, and dry weight of above-ground straw were measured and a harvest index was calculated. The harvest index was a ratio of the grain yield per plant to the above-ground biomass (the sum of the dry weight of above-ground straw and the grain yield per plant).

Measurements of the dry weight of above-ground straw were as follows: after the rice was mature, a single straw with its grains removed was put into a nylon mesh bag and dried to a constant weight at 80°C, and the sample was weighed. 20-30 individual plants were taken from each rice material for repeated tests for statistical analysis.

Measurements of the grain yield were as follows: the grain weight obtained by weighing grains from a single rice plant after ear being threshed and shriveled particles being removed is grain yield per plant. 20-30 individual plants were taken from each rice material for repeated tests for statistical analysis. When the plot yield is calculated, edge row was excluded, and grain weight measured from 30 rice plants of middle rows was taken and calculated as yield of one plot, triplicate plots were used for statistical analysis.

Determination of rice quality was as follows: rice quality was determined after a natural storage for 3 months of the grains of the rice harvested from Beijing field experiment in 2019. The determination was carried out with reference to the industry standard "NY/T 83-2017 Determination of rice quality" under Ministry of Agriculture of the PRC. The results showed a substantial rise in grain yield of the *OsDREB1C* overexpressing rice than that of the wild type, with the difference reaching a significant level. At the same time, its above-ground straw weight was markedly lower than that of the wild type, which enables a significantly increased harvest index compared with the wild type.

According to result of the yield (Table 9), the yield per plant for the transgenic rice was increased by 45.1-67.6% and 7.8-16%, respectively, compared with that for the wild type, in Beijing and Hainan; the yield in the transgenic rice's plots was increased by 41.3-68.3% and 11.9-27.4% than that in the wild type's. At the same time, a decrease of 12.1-24.7% and 17.5-25.8% in the above-ground straw for the transgenic rice compared with the wild type resulted in a significant increase in the harvest index (HI) of the transgenic rice compared with the wild type, with an increase of HI of 10.3-55.7% in Beijing. Meanwhile, the rice quality of the transgenic rice was also elevated, to varying extents, from the wild type (Table 10): the brown rice rate, the milled rice rate and the whole milled rice rate each were significantly higher than that of the wild type, the chalkiness and the chalky grain rate reduced, the appearance quality improved, and the contents of amylose and proteins not significantly affected. Both the yield per plant and the yield per plot for the *OsDREB1C* knockout rice were significantly lower than that of the wild type, while the above-ground biomass was increased, giving direct rise to a substantial decline of 22.4-37.7% in the harvest index compared with the wild type. It showed that introduction of *OsDREB1C* gene into rice can substantially increase the yield, rice quality and harvest index of the transgenic rice. *OsDREB1C* and the coding gene thereof can regulate the grain yield, rice quality and harvest index of rice.

The results for each index are shown in Table 9 and Table 10.

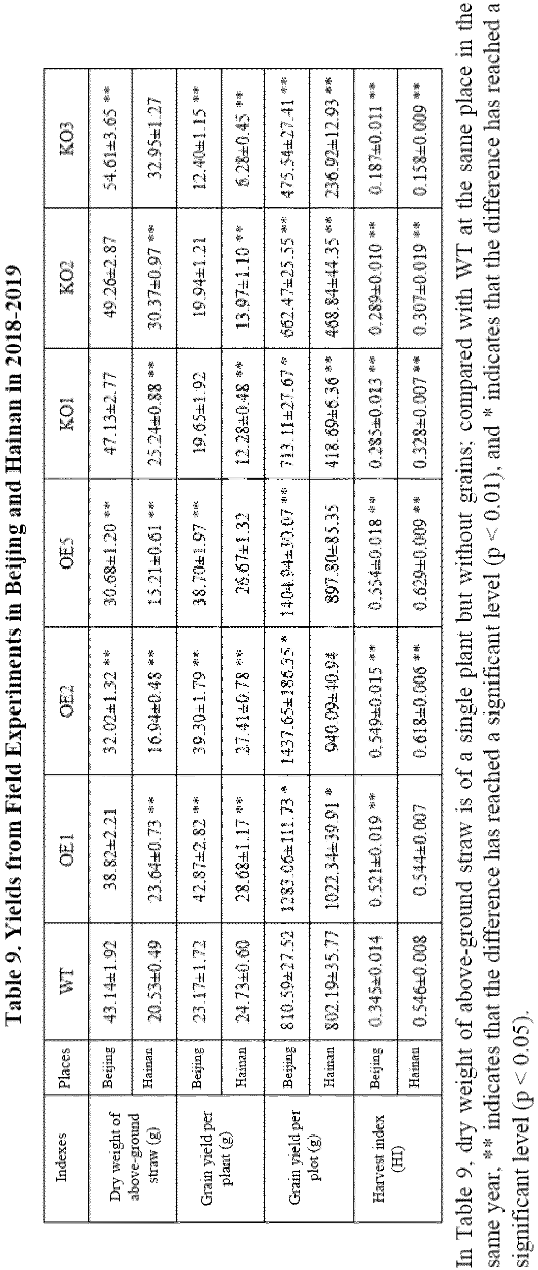

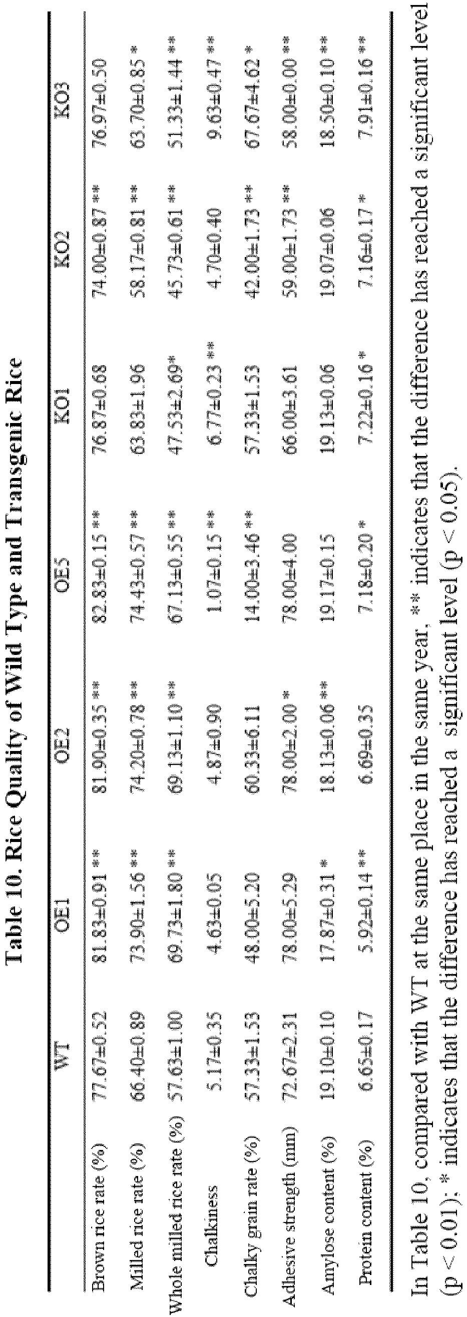

### Example 2. Detection of function of OsDREB1C in wheat (Triticum aestivum) and Arabidopsis thaliana

### 1. Construction of recombinant vector

**Construction of an overexpression vector for wheat:** A full-length of CDS of *OsDREB1C* gene was amplified from cDNA of rice Nipponbare by PCR. The resulting recovered PCR products were linked to the vector backbone of pWMB 110 vector (Huiyun Liu, Ke Wang, Zimiao Jia, Qiang Gong, Zhishan Lin, Lipu Du, Xinwu Pei, Xingguo Ye, Efficient induction of haploid plants in wheat by editing of TaMTL using an optimized Agrobacterium-mediated CRISPR system, Journal of Experimental Botany, Volume 71, Issue 4, 7 February 2020, Pages 1337-1349, https://doi.org/10.1093/jxb/erz529) obtained by double cleaving with BamHI and SacI via In-Fusion Reaction System to give a recombinant vector with correct sequence, designated as pWMB110-*OsDREB1C*. pWMB110-*OsDREB1C* is a recombinant vector obtained by substituting the DNA fragment between BamHI and SacI recognition sites on the pWMB110 vector for the *OsDREB1C* coding gene shown in SEQ ID NO: 2 in the Sequence Listing. pWMB110-*OsDREB1C* can be driven under UBI promoter to express the protein encoded by *OsDREB1C* gene (i.e. the OsDREB1C protein shown in SEQ ID NO: 1).

The primer sequences used are as follows:
Fielder-OsDREB1C-OE-F: 5'-CAGGTCGACTCTAGAGGATCCATGGAGTACTACGAGCAGGAG-3' (SEQ ID NO: 12 in the Sequence Listing);
Fielder-OsDREB1C-OE-R:
   5'-CGATCGGGGAAATTCGAGCTCTCAGTAGCTCCAGAGTGTGAC-3' (SEQ ID NO: 13 in the Sequence Listing).

**Construction of an overexpression vector for *Arabidopsis thaliana:*** CDS of *OsDREB1C* gene (excluding termination codon) was obtained by amplifying from cDNA of rice Nipponbare by PCR. The resulting recovered PCR products were linked to Gateway System Entry Vectors pENTR (ThermoFisher, K240020SP) to give a recombinant vector with correct sequence, designated as pENTR-*OsDREB1C*, which was then in LR reaction with the pGWB5 vector (Nakagawa T, Kurose T, Hino T, Tanaka K, Kawamukai M, Niwa Y, Toyooka K, Matsuoka K, Jinbo T, Kimura T. Development of series of gateway binary vectors, pGWBs, for realizing efficient construction of fusion genes for plant transformation. J Biosci Bioeng. 2007 Jul;104(1):34-41. doi: 10.1263/jbb.104.34.) to transfer the CDS sequence of *OsDREB1C* to the final vector pGWB5, to give a recombinant vector with correct sequence, designated as pGWB5-*OsDREB1C*. pGWB5-*OsDREB1C* can be driven under CaMV 35S promoter to express the protein encoded by *OsDREB1C* gene (i.e. the OsDREB1C protein shown in SEQ ID NO: 1).

The primer sequences used are as follows:
OsDREB1C-CDS-F: 5'-CACCATGGAGTACTACGAGCAGGAG-3' (SEQ ID NO: 14 in the Sequence Listing);
OsDREB1C-CDS-R: 5'-GTAGCTCCAGAGTGTGACGTC-3' (SEQ ID NO: 15 in the Sequence Listing).

### 2. Construction of a transgenic plant

After disinfecting the immature embryo of Fielder, a cultivar of wheat, it was induced to provide embryogenic callus. pWMB110-*OsDREB1C* obtained in step 1 was introduced into *Agrobacterium* C58C1. The callus was transfected and co-cultured with *Agrobacterium* using a genetic transformation method for wheat, mediated by *Agrobacterium,* to provide a transgenic plant upon resistance screening. The screened transgenic plant was an *OsDREB1C* transgenic wheat material. Due to the extremely high similarity between *OsDREB1C* and homologous genes of wheat's own, it failed to detect the expression level of *OsDREB1C* gene in wheat by qRT-PCR assay. Therefore, the presence or absence of Bar gene of the vector in the cDNA of the transgenic wheat was examined by PCR assay using wild-type Fielder as a control. The primers used were as follows: 5'-CAGGAACCGCAGGAGTGGA-3' (SEQ ID NO: 16 in the Sequence Listing), 5'-CCAGAAACCCACGTCATGCC-3' (SEQ ID NO: 17 in the Sequence Listing). Electrophoresis detection showed that Bar gene was present in all three lines (TaOE-5, TaOE-8 and TaOE-9) of the *OsDREB1C* transgenic wheat, but not in the wild type, indicating that the three lines were all a transgenic material introduced with the pWMB110*-OsDREB1C* vector (A of Fig. 5).

The pWMB110*-OsDREB1*C obtained in step 1 was introduced into *Agrobacterium* GV3101, followed by transferring to Col-0, a wild-type *Arabidopsis thaliana,* using an *Agrobacterium*-mediated floral dip method. After being harvested the seeds, they were seeded into resistant media for screening to provide a transgenic plant, that is, an *OsDREB1C* transgenic *Arabidopsis thaliana* material. The relative expression level of *OsDREB1C* gene at RNA level in the *OsDREB1C* transgenic *Arabidopsis thaliana* was detected by qRT-PCR assay, using Col-0, a wild-type *Arabidopsis thaliana,* as a control. The primers used were as follows: 5'-CATGATGATGCAGTACCAGGA-3' (SEQ ID NO: 18 in the Sequence Listing), 5'-GATCATCAGTAGCTCCAGAGTG-3' (SEQ ID NO: 19 in the Sequence Listing); the internal reference gene is *Arabidopsis thaliana* Actin, and the primers for the internal reference gene are: 5'-GCACCACCTGAAAGGAAGTACA -3' (SEQ ID NO: 20 in the Sequence Listing), 5'-CGATTCCTGGACCTGCCTCATC -3' (SEQ ID NO: 21 in the Sequence Listing). The results showed that the relative expression levels of *OsDREB1C* gene in the three lines of *OsDREB1C* transgenic *Arabidopsis thaliana* (AtOE-10, AtOE-11 and AtOE-12) were significantly higher than that in the wild type (Col-0), and the three lines were all an *OsDREB1*overexpressing *Arabidopsis thaliana* material (B of Fig. 5).

### 3. Phenotypic identification of the OsDREB1C transgenic wheat

Pot grown wheat in a greenhouse was identified for its phenotype. The materials to be tested: wild-type wheat (Fielder) and *OsDREB1C* overexpressing wheat (TaOE-5, TaOE-8 and TaOE-9). The greenhouse temperature was controlled at 22-24°C, and the length of day was 12 hours of light /12 hours of darkness.

The heading stages of wild-type and transgenic wheat were counted, and the photosynthetic rates of the flag leaves of the wheat to be tested at the heading stages were measured using LICOR-6400XT Portable Photosynthesis System (LI-COR, USA), with light intensity set at 1000 µmοl m⁻² s⁻¹. The grain number, 1000-grain weight, and grain yield per plant were measured after wheat grains went mature.

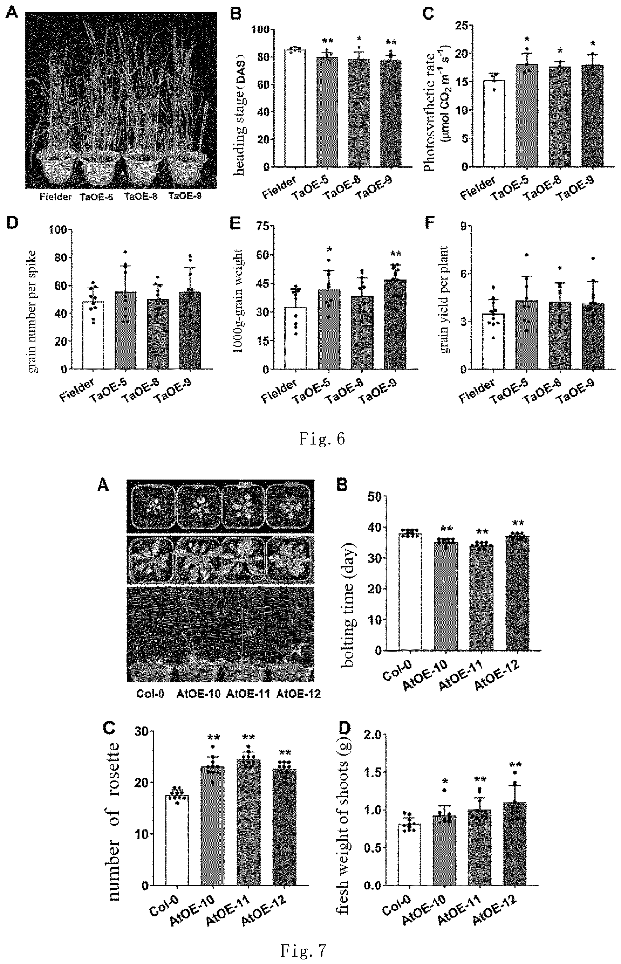

The results showed (Table 11 and Fig. 6) that the transgenic wheat (TaOE-5, TaOE-8 and TaOE-9) spiked earlier than the wild-type Fielder while having a higher photosynthetic rate than the wild type. The grain number per ear among the yield traits was also higher than that of the wild type, and the 1000-grain weight was significantly larger than that of the wild type, resulting in an increase in yield per plant ultimately, indicating that OsDREB1C likewise functions in promoting heading date, improving photosynthetic capacity and increasing yield in wheat. The heading stage was the days from sowing to heading.

**Table 11. Heading Stage, Photosynthetic Rate and Yield Traits of Wild-Type and Transgenic Wheat**

| Wheat | Heading Stage (Days) | Photosynthetic Rate (µmol CO₂ m⁻¹ s⁻¹) | Grain Number | 1000-Grain Weight (g) | Yield per Plant (g) |
|---|---|---|---|---|---|
| Fielder | 85.17±1.47 | 15.27±1.23 | 48.5±9.72 | 32.53±9.49 | 3.48±0.88 |
| TaOE-5 | 80±2.96 | 18.11±1.87 | 55.1±18.62 | 41.89±9.75 | 4.32±1.50 |
| TaOE-8 | 78.43±5.03 | 17.68±0.89 | 50.27±10.15 | 38.39±9.47 | 4.24±1.18 |
| TaOE-9 | 77.5±3.50 | 17.96±1.81 | 55.2±17.31 | 46.89±7.61 | 4.15±1.33 |

### 4. Phenotypic identification of the OsDREB1C transgenic Arabidopsis thaliana

*Arabidopsis thaliana* grown in a greenhouse was identified for its phenotype. The materials to be tested: wild-type *Arabidopsis thaliana* (Col-0) and *OsDREB1C* overexpressing *Arabidopsis thaliana* (AtOE-10, AtOE-11 and AtOE-12). They were grown in short day (8 hours of light /16 hours of darkness) for 2 weeks, and then transferred to long day condition (16 hours of light /8 hours of darkness).

The bolting stage, number of rosette leaves, and above-ground biomass (comprising fresh weight and dry weight) were counted for wild-type and transgenic *Arabidopsis thaliana.* Bolting stage is the number of days from sowing to stem elongation and flowering.

The results showed (Table 12 and Fig. 7) that the transgenic *Arabidopsis thaliana* (AtOE-10, AtOE-11 and AtOE-12) bolted earlier (1-3.9 days) than wild-type Col-0, with 5-7 more rosette leaves than the wild type when bolting, and their above-ground fresh weight and dry weight were both higher than the wild type, indicating that overexpression of *OsDREB1C* can likewise increase the biomass of a dicotyledon plant, *Arabidopsis thaliana,* and enable *Arabidopsis thaliana* bolt earlier.

**Table 12. Phenotypic Indexes of Wild-Type and Transgenic Arabidopsis thaliana**

| *Arabidopsis Thaliana* | Bolting Stage (days) | Number of Rosette Leaves | Fresh Weight (g) |
|---|---|---|---|
| Col-0 | 38±0.94 | 17.6±0.97 | 0.81±0.08 |
| AtOE-10 | 35.1±0.99 | 23.1±1.91 | 0.93±0.12 |
| AtOE-11 | 34.1±0.74 | 24.6±1.26 | 1.01±0.14 |
| AtOE-12 | 37±0.82 | 22.6±1.35 | 1.10±0.20 |

### Example 3: Haplotypes Hap.1, Hap.2 and Hap.3 of rice are correlated with yield of rice

709 rice core germplasm materials, including 299 *indica,* 355 temperate *japonica,* 14 tropical *japonica,* and 41 intermediate were for testing.

Each of the materials for testing was planted, followed by counting and recording grain yield per plant after they were mature.

A literature (Li et al., Analysis of genetic architecture and favorable allele usage of agronomic traits in a large collection of Chinese rice accessions, SCIENCE CHINA Life Sciences, November 2020, Vol. 63 No. 11: 1688 - 1702, https://doi.org/10.1007/s11427-019-1682-6) had disclosed the sequences of 709 rice core germplasm materials (the sequence of each rice material disclosed in this literature was in NCBI, BioProject PRJCA000322 and GSA accession CRA000167, website: https://bigd.big.ac.cn/), based on which alignment was performed for SNP genotypes among populations, and the SNP of the first 2kbs in the promoter region of the gene LOC_Os06g03670 was taken in the vcf file. According to the results of GWAS, missense mutation(s) in the gene region and site(s) with -Log10 (P-value) values greater than 10 in the promoter region served as SNPs for distinguishing haplotypes. General Linear Model in the software TASSEL 5.0 (http://www.maizegenetics.net/tassel) as developed by the Buckler Laboratory of Cornell University (Zhang Z, Ersoz E, Lai CQ, Todhunter RJ, Tiwari HK, Gore MA, Bradbury PJ, Yu J, Arnett DK, Ordovas JM, Buckler ES. Mixed linear model approach adapted for genome-wide association studies. Nature Genetics. 2010 Apr;42(4):355-60. https://doi.org/10.1038/ng.546) was employed for the analysis of correlation of SNPs with grain yield per plant.

The results showed that three haplotypes, Hap.1, Hap.2 and Hap.3, respectively, of this DNA fragment in rice genome were found to be related to grain yield per plant. The three haplotypes involved 10 SNP sites, whose physical positions in the reference genome (version number: Os-Nipponbare-Reference-IRGSP-1.0 (IRGSP-1.0), updated on February 6, 2013, website: https://rapdb.dna.affrc.go.jp/) were, in sequence, 1433007, 1433155, 1433229, 1433365, 1433528, 1433919, 1434216, 1434258, 1434486, and 1434806, as shown in Figure 8 for their positions on the first 2kbs in the promoter of the gene LOC_Os06g03670. The nucleotides of these 10 sites for Hap.1 were, in sequence, A, A, G, C, A, G, G, C, T, and G; the nucleotides of these 10 sites of Hap.2 were, in sequence, A, G, G, C, T, G, G, C, T, and G; and the nucleotides of these 10 sites of Hap. 3 were, in sequence, G, G, A, G, T, A, A, T, G, and T. Haplotypes of each rice material were shown in Tables 13 and 14.

Grain yield per plant was counted for the three haplotypes, and results for some of the rice materials were shown in Tables 13 and 14.

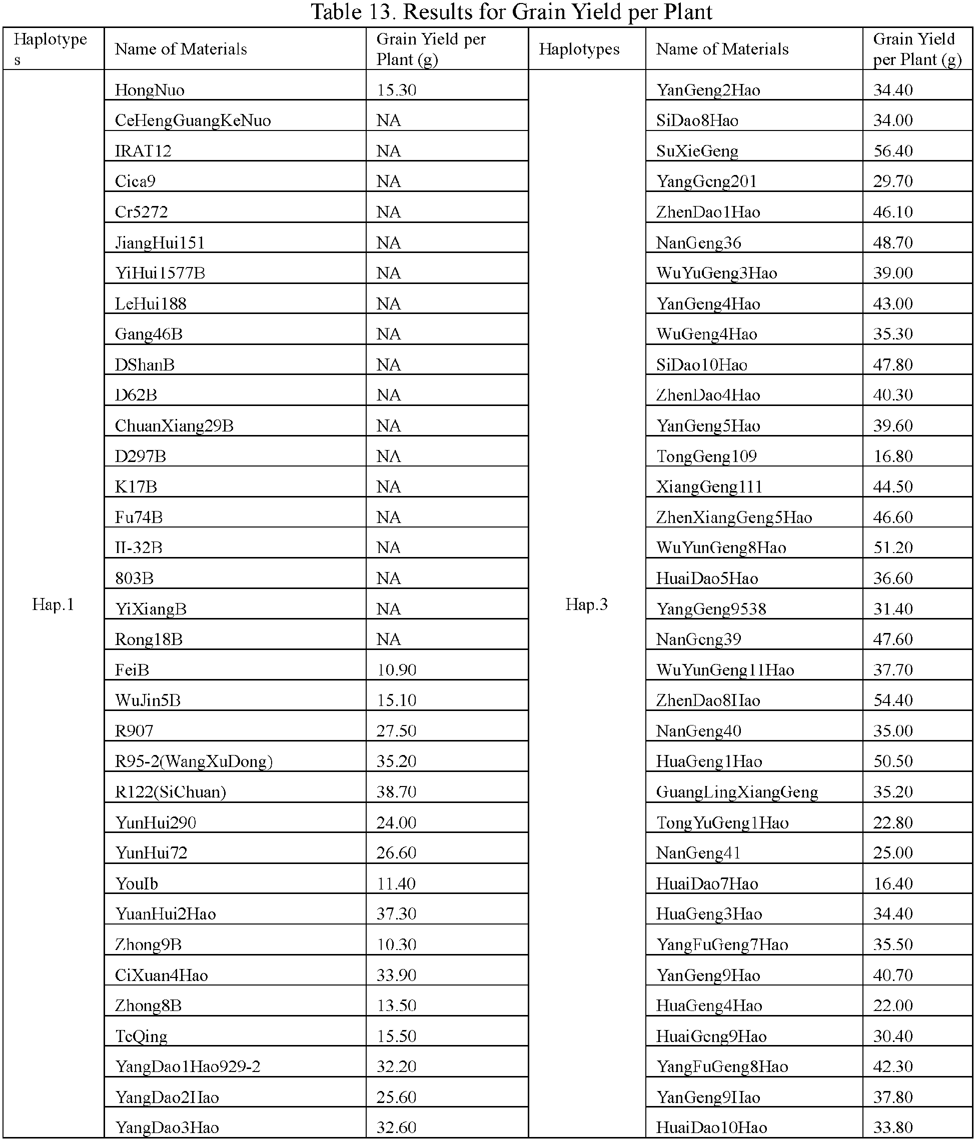

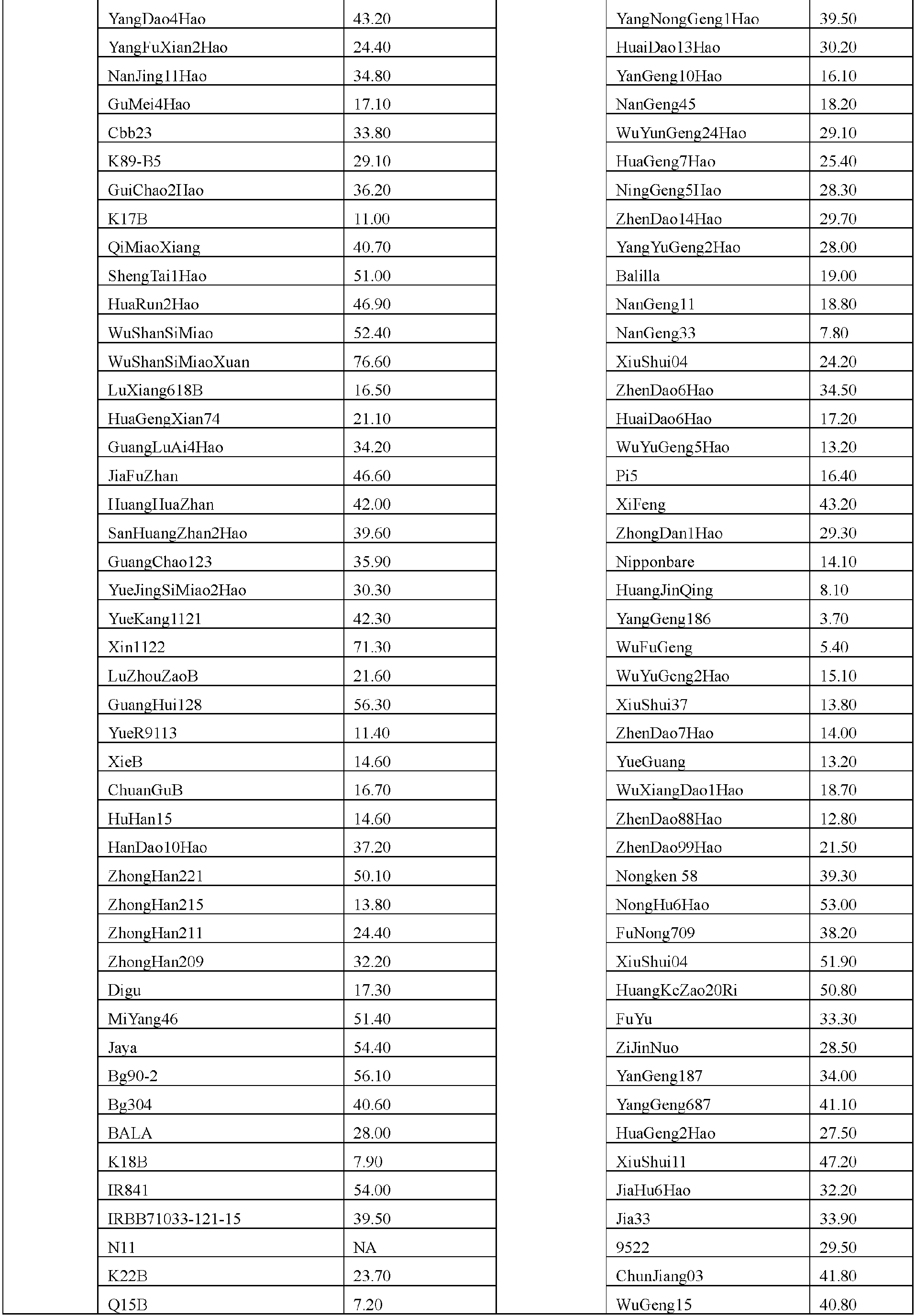

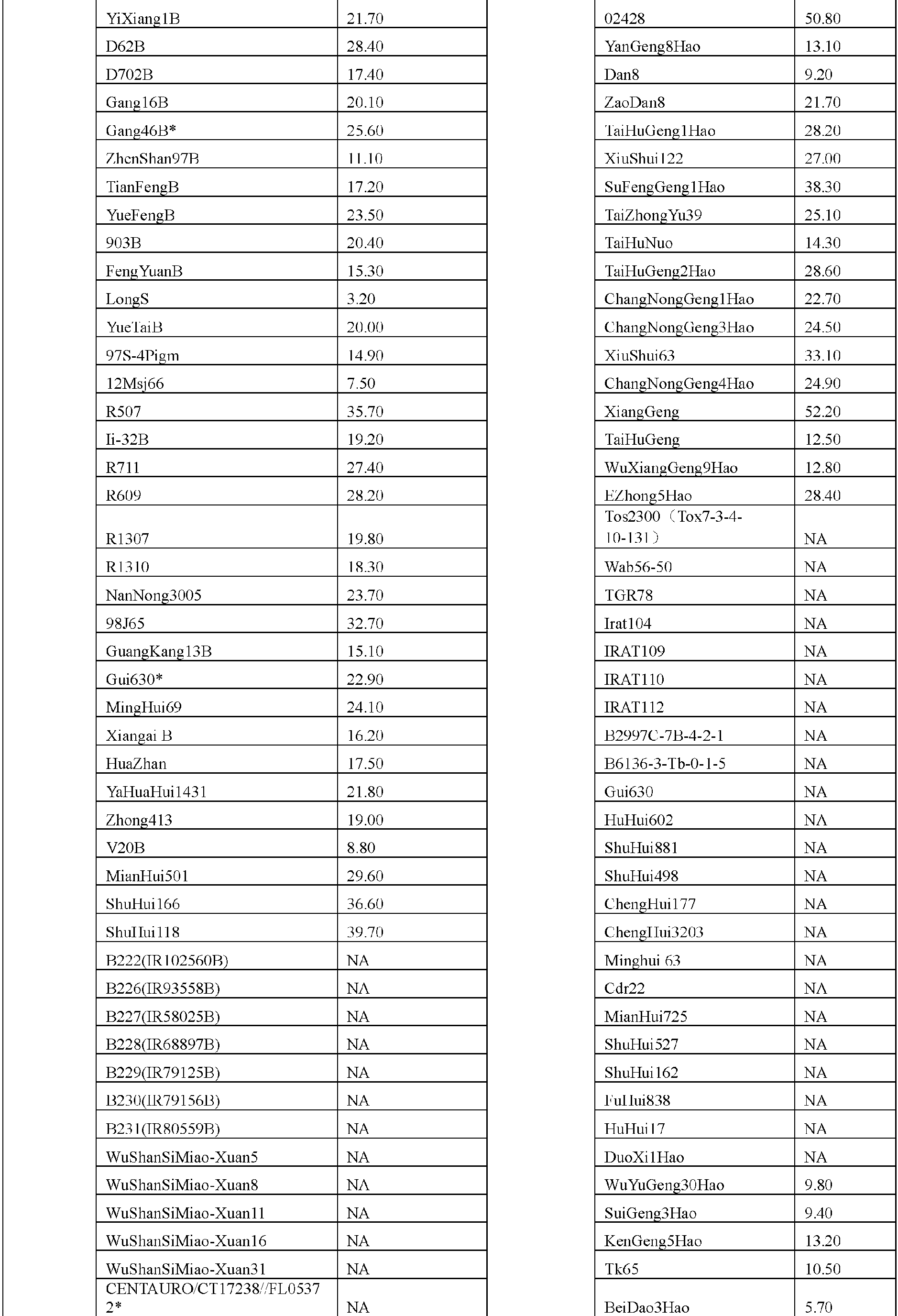

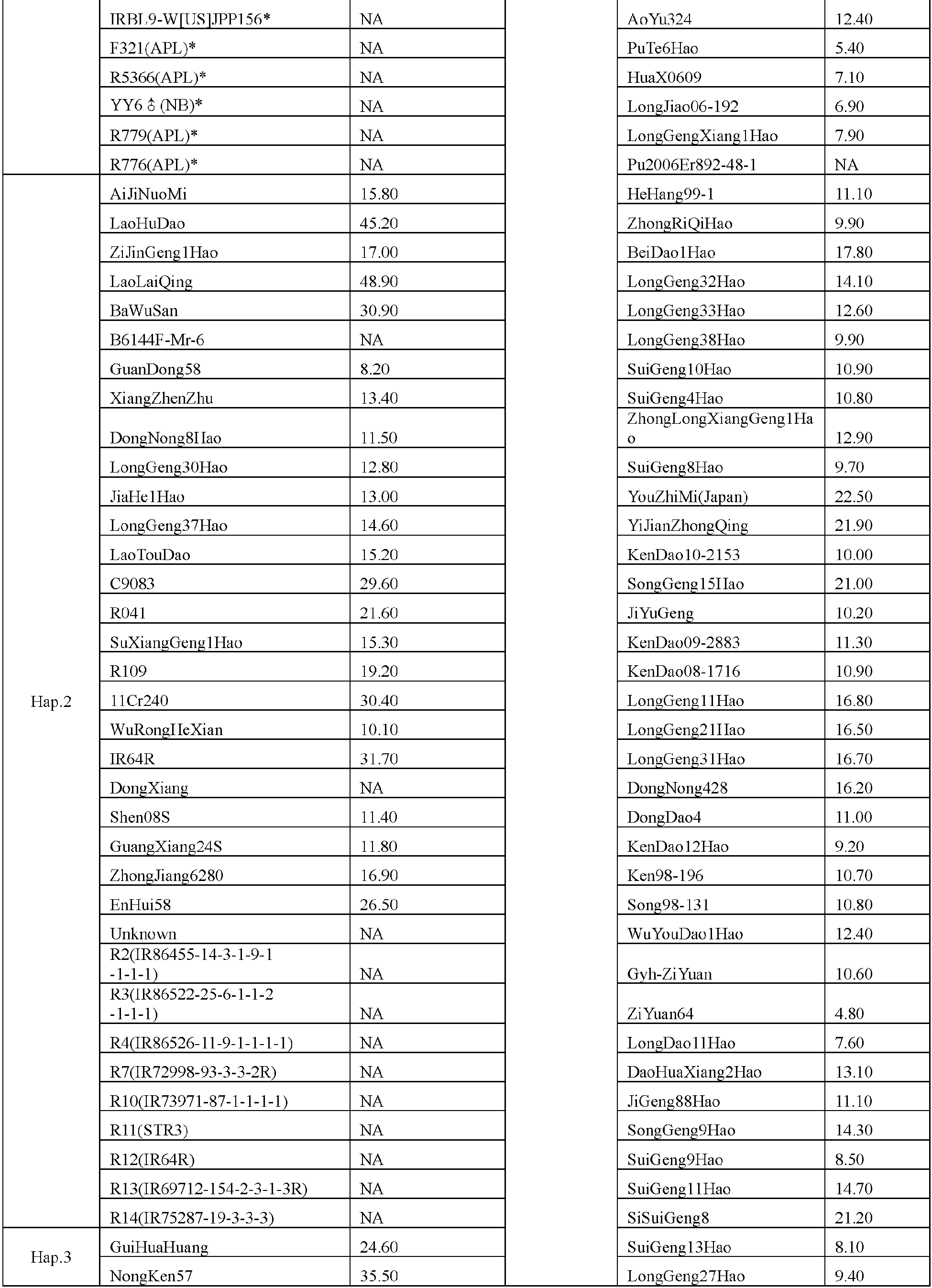

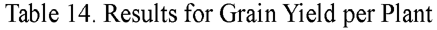

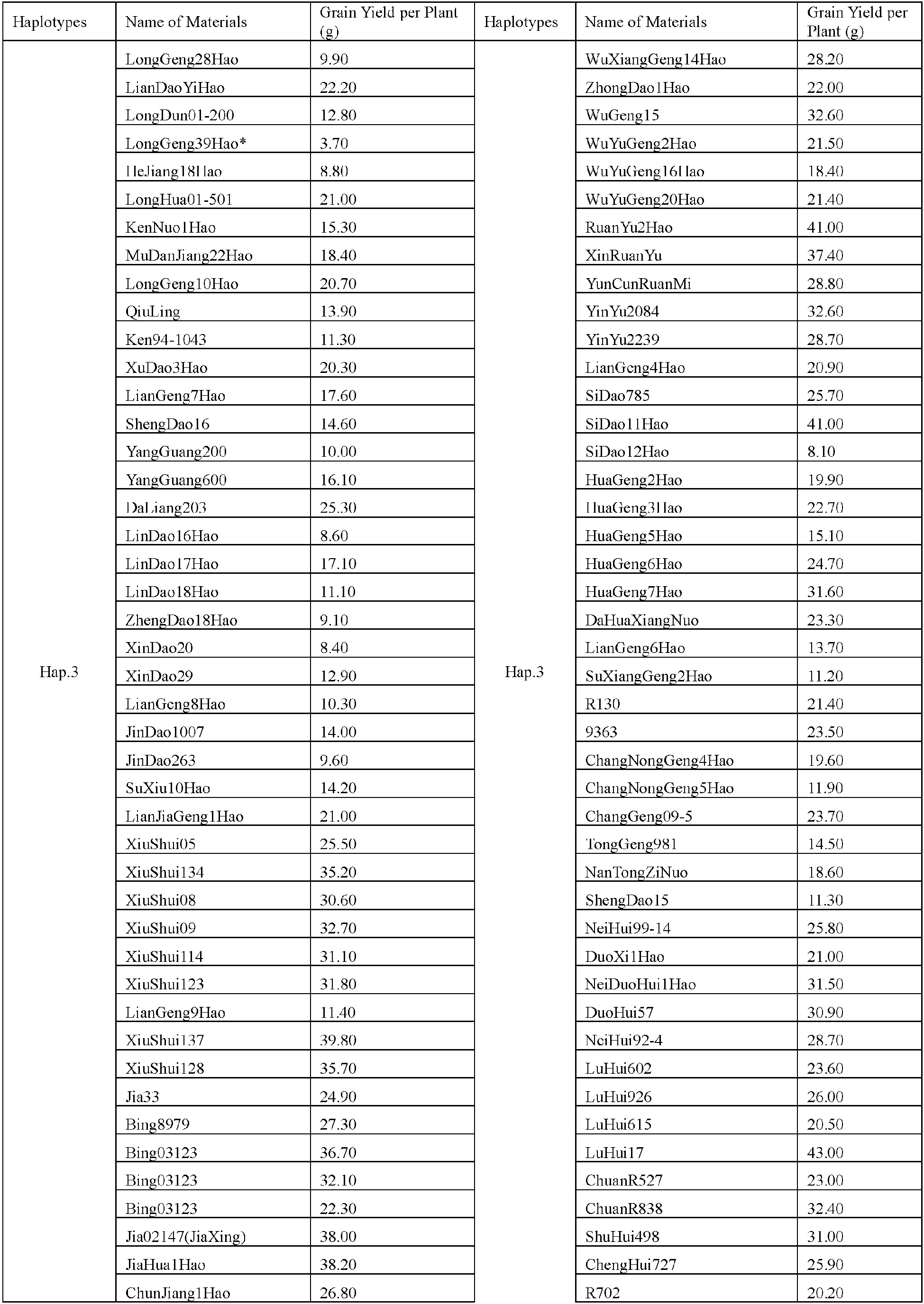

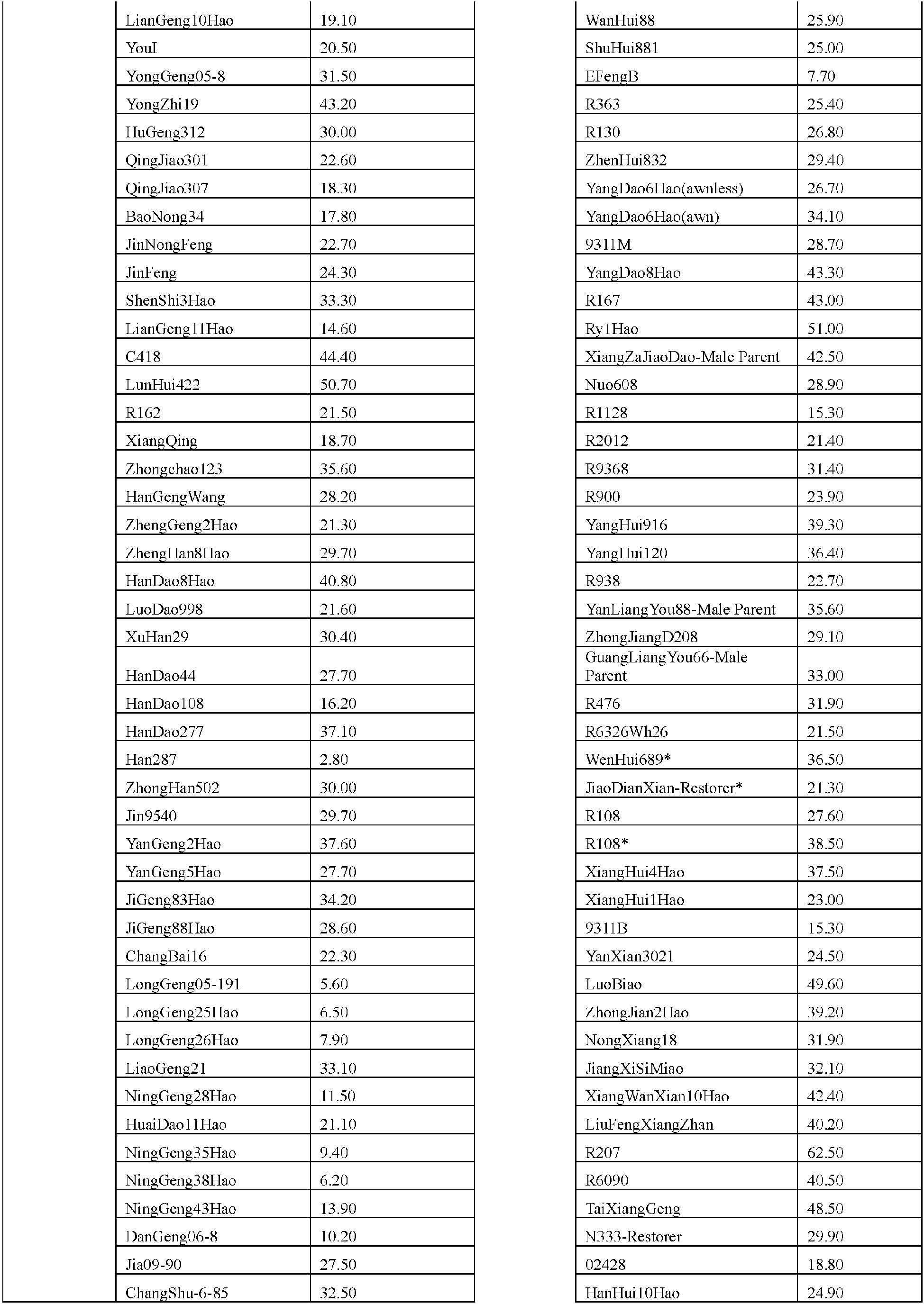

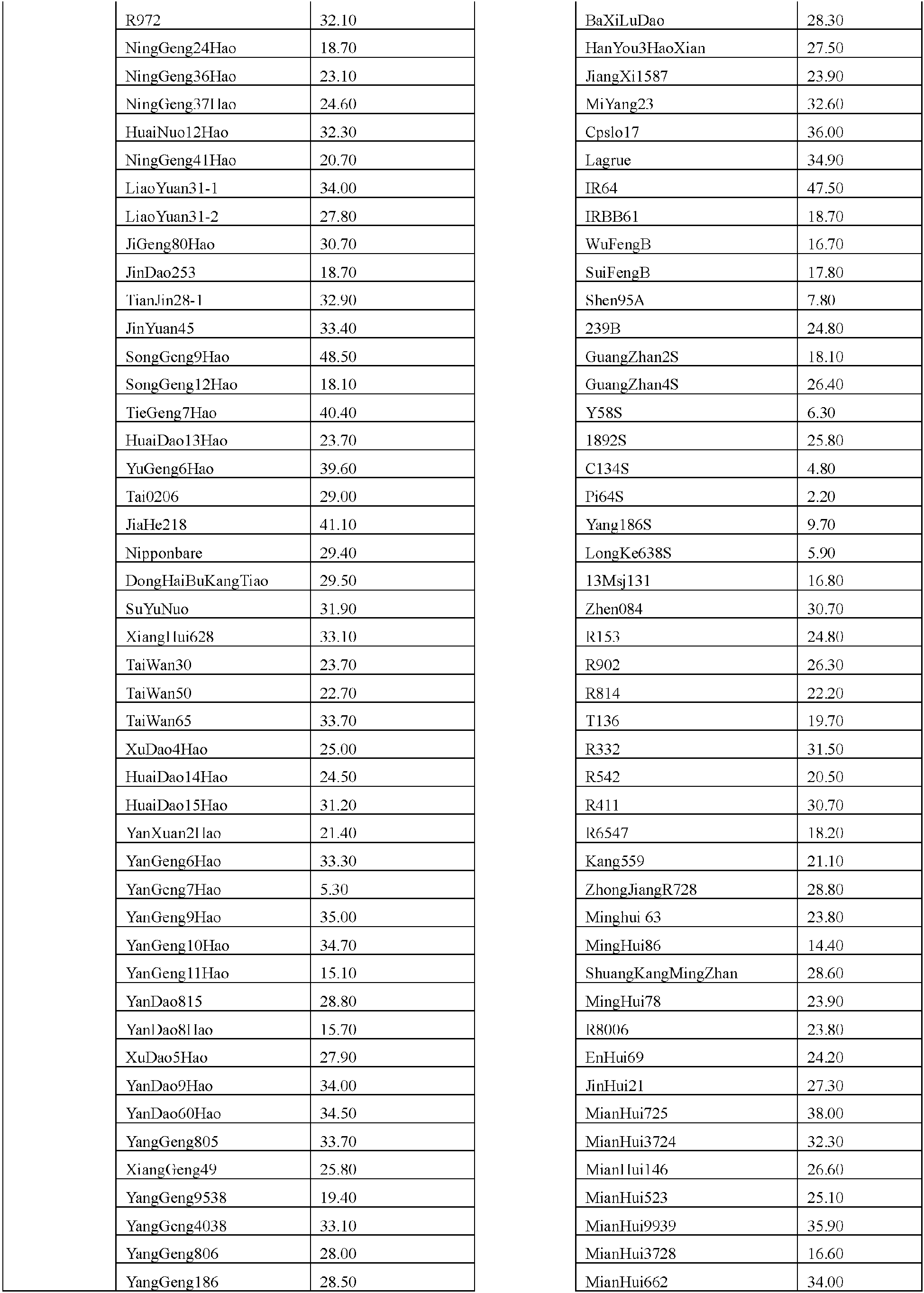

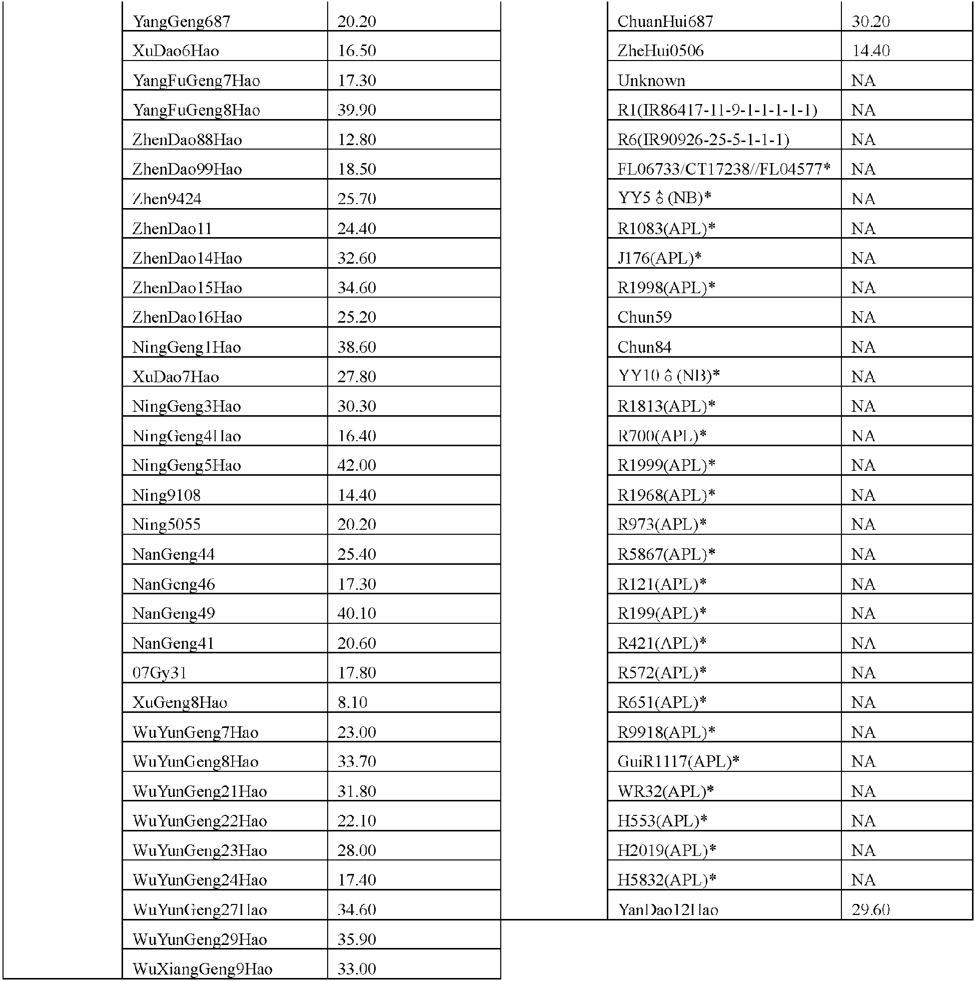

In Tables 13 and 14, NA represents the absence of data.

Grain yield per plant was counted for each of the haplotypes. The grain yield per plant of the rice having Hap. 1 as haplotype was 27.98 ± 1.49g, the grain yield per plant of the rice having Hap.2 as haplotype was 20.48 ± 2.30g, and the grain yield per plant of the rice having Hap.3 as haplotype was 25.23 ± 0.53g; the grain yield per plant of the rice having Hap.2 as haplotype was significantly lower than that having Hap.3 as haplotype, and there was no significant differences in grain yield per plant between the rice having Hap. 1 as haplotype, and both the rice having Hap.2 and Hap.3 as haplotypes (Fig. 9), indicating that the three haplotypes of rice were related to grain yield per plant of rice, and could be used in rice breeding.

The invention has been described in detail above. For those skilled in the art, the invention can be carried out within a wider range at equivalent parameters, concentrations and conditions, without departing from the purpose and scope of the invention and without unnecessary experiments. Although the invention provides specific examples, it is to be appreciated that the invention can be further modified. In short, according to the principle of the invention, the application is intended to include any modifications, uses or improvements to the invention, including changes made with conventional techniques known in the art beyond the scope contained in the application. Some fundamental features can be applied, according to the scope of the following appended claims.

### Industrial application

Experiments have demonstrated that OsDREB1C and the associated biological materials thereof of the invention can enhance the photosynthetic efficiency of a plant, promote nitrogen uptake and transport and increase the nitrogen content in the plant and its grains, promote earlier heading, and improve yield. Aiming at a synergetic enhancement in photosynthetic efficiency, nitrogen use efficiency and heading stage of crops, the invention has achieved a synergetic increase in nitrogen use efficiency while yielding a substantial amount of crops, and also provided a solution to the contradiction between high yield and prematurity of crops. Therefore, the invention further strengthens the potential in substantially yielding crops and increases nitrogen use efficiency, achieving "high yield and high efficiency"; on the other hand, the solution of the contradiction between high yield and prematurity has found potential applications to dealing with prematurity and high yield of direct seeded rice and, grains and cash crops, grains and vegetables, continuous cropping rice for grains and oil, double cropping rice, as well as "transgressive late maturing" of inter-subspecies hybrid rice, etc.
In addition, the invention starts from the function of *OsDREB1C* gene in regulating yield of rice to analyze the haplotypes of its gene sequence, and discovers that haplotype 3 (Hap. 3) has excellent traits that increases yield of rice, and finds bright prospects in application to genetic breeding of rice and cultivar modification.

## Claims

1. Any of the following uses of a protein, or a material that regulates the activity or content of the protein:
D1) regulation of yield of a plant;
D2) preparation of a product that regulates yield of a plant;
D3) regulation of photosynthesis and yield of a plant;
D4) preparation of a product that regulates photosynthesis, and yield of plant;
D5) regulation of nitrogen uptake or transport in, photosynthesis and yield of a plant;
D6) preparation of a product that regulates nitrogen uptake or transport in, photosynthesis and yield of a plant;
D7) regulation of nitrogen content in, photosynthesis and yield of a plant;
D8) preparation of a product that regulates nitrogen content in, photosynthesis and yield of a plant;
D9) regulation of nitrogen uptake or transport in, photosynthesis, blooming stage, and yield of a plant;
D10) preparation of a product that regulates nitrogen uptake or transport in, photosynthesis, blooming stage and yield of a plant;
D11) regulation of nitrogen content in, photosynthesis, blooming stage and yield of a plant;
D12) preparation of a product that regulates nitrogen content in, photosynthesis, blooming stage and yield of a plant;
D13) breeding of a plant;
the protein is A1), A2), A3) or A4), as follows:
A1) a protein having an amino acid sequence of SEQ ID NO: 1;
A2) a protein obtained by subjecting the amino acid sequence shown in SEQ ID NO: 1 in the Sequence Listing to substitution and/or deletion and/or addition of one or more amino acid residues and having the same function;
A3) a protein derived from rice, maize, sorghum, soybean, *Brassica napus* L., Arabidopsis, millet, aegilops, *Brachypodium distachyon,* or wheat, and having 64% or more identity with SEQ ID NO: 1 and having the same function as the protein of A1);
A4) a fusion protein obtained by linking a tag to the N-terminus or/and C-terminus of A1) or A2) or A3).

2. The use according to claim 1, **characterized in that**, the plant is M1) or M2) or M3):
M1) a monocotyledon, or dicotyledon plant;
M2) a gramineous plant, a cruciferous plant, or a leguminous plant;
M3) rice, wheat, maize, Arabidopsis, *Brassica napus* L., or soybean.

3. The use according to claim 1 or 2, **characterized in that**, the photosynthesis is reflected in photosynthetic rate, net photosynthetic rate, heat dissipation NPQ, maximum carboxylation efficiency, and/or maximum electron transfer rate;
the transport is reflected in a transport from roots to above-ground parts, or to grains;
the nitrogen content is nitrogen content in a plant or in an organ;
the blooming stage is reflected in heading stage;
the yield is reflected in grain yield, above-ground yield, and/or harvest index.

4. Any of the following uses of a biological material associated with the protein of claim 1:
D1) regulation of yield of a plant;
D2) preparation of a product that regulates yield of a plant;
D3) regulation of photosynthesis, and yield of a plant;
D4) preparation of a product that regulates photosynthesis, and yield of plant;
D5) regulation of nitrogen uptake or transport in, photosynthesis and yield of a plant;
D6) preparation of a product that regulates nitrogen uptake or transport in, photosynthesis and yield of a plant;
D7) regulation of nitrogen content in, photosynthesis and yield of a plant;
D8) preparation of a product that regulates nitrogen content in, photosynthesis and yield of a plant;
D9) regulation of nitrogen uptake or transport in, photosynthesis, blooming stag and yield of a plant;
D10) preparation of a product that regulates nitrogen uptake or transport in, photosynthesis, blooming stage and yield of a plant;
D11) regulation of nitrogen content in, photosynthesis, blooming stage and yield of a plant;
D12) preparation of a product that regulates nitrogen content in, photosynthesis, blooming stage and yield of a plant;
D13) breeding of a plant;
the biological material is any of B1) to B9), as follows:
B1) a nucleic acid molecule encoding the protein of claim 1;
B2) an expression cassette containing the nucleic acid molecule of B1);
B3) a recombinant vector containing the nucleic acid molecule of B1), or a recombinant vector containing the expression cassette of B2);
B4) a recombinant microorganism containing the nucleic acid molecule of B1), or a recombinant microorganism containing the expression cassette of B2), or a recombinant microorganism containing the recombinant vector of B3);
B5) a transgenic plant cell line containing the nucleic acid molecule of B1), or a transgenic plant cell line containing the expression cassette of B2);
B6) a transgenic plant tissue containing the nucleic acid molecule of B1), or a transgenic plant tissue containing the expression cassette of B2);
B7) a transgenic plant organ containing the nucleic acid molecule of B1), or a transgenic plant organ containing the expression cassette of B2);
B8) a nucleic acid molecule for reducing the expression of the protein of claim 1, or with coding gene of the protein of claim 1 knocked out;
B9) an expression cassette, a recombinant vector, a recombinant microorganism, a transgenic plant cell line, a transgenic plant tissue, or a transgenic plant organ containing the nucleic acid molecule of B8).

5. The use according to claim 4, **characterized in that**, the nucleic acid molecule of B1) is b11) or b12) or b13) or b14) or b15), as follows:
b11) a cDNA or DNA molecule having a coding sequence of SEQ ID NO: 2 in the Sequence Listing;
b12) a cDNA or DNA molecule shown in SEQ ID NO: 2 in the Sequence Listing;
b13) a DNA molecule shown in SEQ ID NO: 3 at positions 3001-4131 in the Sequence Listing;
b14) a cDNA or DNA molecule having 73% or more identity with the nucleotide sequence defined in b11) or b12) or b13), and encoding the protein of claim 1;
b15) a cDNA or DNA molecule hybridizing with the nucleotide sequence defined in b11) or b12) or b13)or b14) under stringent conditions, and encoding the protein of claim 1;
the expression cassette of B2) is b21) or b22) or b23), as follows:
b21) a DNA molecule shown in SEQ ID NO: 3 in the Sequence Listing;
b22) a DNA molecule having 73% or more identity with, and the same function as the nucleotide sequence defined in b21);
b23) a DNA molecule hybridizing, under stringent conditions, with and having the same function as the nucleotide sequence defined in b21) or b22).

6. The use according to claim 4 or 5, **characterized in that**, the plant is M1) or M2) or M3):
M1) a monocotyledon, or dicotyledon plant;
M2) a gramineous plant, a cruciferous plant, or a leguminous plant;
M3) a rice, a wheat, a maize, an Arabidopsis, a *Brassica napus* L., or a soybean.

7. The use according to claim 4 or 5, **characterized in that**, the photosynthesis is reflected in photosynthetic rate, net photosynthetic rate, heat dissipation NPQ, maximum carboxylation efficiency, and/or maximum electron transfer rate;
the transport is reflected in a transport from roots to above-ground parts, or to grains;
the nitrogen content is nitrogen content in a plant or in an organ;
the blooming stage is reflected in heading stage;
the yield is reflected in grain yield, above-ground yield, and/or harvest index.

8. The use according to claim 4 or 5, **characterized in that**, the plant is M1) or M2) or M3):
M1) a monocotyledon, or dicotyledon plant;
M2) a gramineous plant, a cruciferous plant, or a leguminous plant;
M3) rice, wheat, maize, Arabidopsis, *Brassica napus* L., or soybean;
the photosynthesis is reflected in photosynthetic rate, net photosynthetic rate, heat dissipation NPQ, maximum carboxylation efficiency, and/or maximum electron transfer rate;
the transport is reflected in a transport from roots to above-ground parts, or to grains;
the nitrogen content is nitrogen content in a plant or in an organ;
the blooming stage is reflected in heading stage;
the yield is reflected in grain yield, above-ground yield, and/or harvest index.

9. Any of the methods as follows:
X1) a method for breeding a plant with increased yield, comprising expressing the protein of claim 1 in a recipient plant, or increasing the content or activity of the protein of claim 1 in the recipient plant to obtain a target plant with increased yield;
X2) a method for breeding a plant with decreased yield, comprising lowering the content or activity of OsDREB1C in a recipient plant, or inhibiting the expression of OsDREB1C coding gene in the recipient plant to obtain a target plant with decreased yield compared to the recipient plant;
X3) a method for breeding a plant with enhanced photosynthesis and increased yield, comprising expressing the protein of claim 1 in a recipient plant, or increasing the content or activity of the protein of claim 1 in the recipient plant to obtain a target plant with enhanced photosynthesis and increased yield;
X4) a method for breeding a plant with enhanced photosynthesis, enhanced nitrogen uptake or transport capability, and increased yield, comprising expressing the protein of claim 1 in a recipient plant, or increasing the content or activity of the protein of claim 1 in the recipient plant to obtain a target plant with enhanced photosynthesis, enhanced nitrogen uptake or transport capability, and increased yield;
X5) a method for breeding a plant with enhanced photosynthesis, increased nitrogen content, and increased yield, comprising expressing the protein of claim 1 in a recipient plant, or increasing the content or activity of the protein of claim 1 in the recipient plant to obtain a target plant with enhanced photosynthesis, increased nitrogen content, and increased yield;
X6) a method for breeding a plant with enhanced photosynthesis, enhanced nitrogen uptake or transport capability, earlier blooming stage, and increased yield, comprising expressing the protein of claim 1 in a recipient plant, or increasing the content or activity of the protein of claim 1 in the recipient plant to obtain a target plant with enhanced photosynthesis, enhanced nitrogen uptake or transport capability, earlier blooming stage, and increased yield;
X7) a method for breeding a plant with enhanced photosynthesis, increased nitrogen content, earlier blooming stage, and increased yield, comprising expressing the protein of claim 1 in a recipient plant, or increasing the content or activity of the protein of claim 1 in the recipient plant to obtain a target plant with enhanced photosynthesis, increased nitrogen content, earlier blooming stage, and increased yield.

10. The method according to claim 9, **characterized in that**, the method of X1) - X7) is realized by introducing the coding gene of the protein of claim 1 into the recipient plant and expressing the coding gene.

11. The method according to claim 10, **characterized in that**, the coding gene is the nucleic acid molecule of B1) of claim 4 or 5.

12. The method according to any one of claims 9-11, **characterized in that**, the plant is M1) or M2) or M3):
M1) a monocotyledon, or dicotyledon plant;
M2) a gramineous plant, a cruciferous plant, or a leguminous plant;
M3) rice, wheat, maize, Arabidopsis, *Brassica napus* L., or soybean.

13. The method according to any of claims 9-11, **characterized in that**, the photosynthesis is reflected in photosynthetic rate, net photosynthetic rate, heat dissipation NPQ, maximum carboxylation efficiency, and/or maximum electron transfer rate;
the transport is reflected in a transport from roots to above-ground parts, or to grains;
the nitrogen content is nitrogen content in a plant or in an organ;
the blooming stage is reflected in heading stage;
the yield is reflected in grain yield, above-ground yield, and/or harvest index.

14. The method according to any of claims 9-11, **characterized in that**, the plant is M1) or M2) or M3):
M1) a monocotyledon, or dicotyledon plant;
M2) a gramineous plant, a cruciferous plant, or a leguminous plant;
M3) rice, wheat, maize, Arabidopsis, *Brassica napus* L., or soybean;
the photosynthesis is reflected in photosynthetic rate, net photosynthetic rate, heat dissipation NPQ, maximum carboxylation efficiency, and/or maximum electron transfer rate;
the transport is reflected in a transport from roots to above-ground parts, or to grains;
the nitrogen content is nitrogen content in a plant or in an organ;
the blooming stage is reflected in heading stage;
the yield is reflected in grain yield, above-ground yield, and/or harvest index.

15. A product having any of the following functions D1) - D6), comprising the protein of claim 1 or the biological material of claim 4 or 5:
D1) regulation of yield of a plant;
D2) regulation of photosynthesis and yield of a plant;
D3) regulation of nitrogen uptake or transport in, photosynthesis and yield of a plant;
D4) regulation of nitrogen content in, photosynthesis and yield of a plant;
D5) regulation of nitrogen uptake or transport in, photosynthesis, blooming stage and yield of a plant;
D6) regulation of nitrogen content in, photosynthesis, blooming stage and yield of a plant.

16. The product according to claim 15, **characterized in that**, the plant is M1) or M2) or M3):
M1) a monocotyledon, or dicotyledon plant;
M2) a gramineous plant, a cruciferous plant, or a leguminous plant;
M3) rice, wheat, maize, Arabidopsis, *Brassica napus* L., or soybean.

17. The product according to claim 15 or 16, **characterized in that**, the photosynthesis is reflected in photosynthetic rate, net photosynthetic rate, heat dissipation NPQ, maximum carboxylation efficiency, and/or maximum electron transfer rate;
the transport is reflected in a transport from roots to above-ground parts, or to grains;
the nitrogen content is nitrogen content in a plant or in an organ;
the blooming stage is reflected in heading stage;
the yield is reflected in grain yield, above-ground yield, and/or harvest index.

18. A method for detecting yield traits of rice, comprises: detecting haplotypes of rice to be tested, the haplotypes of the rice being Hap.1, Hap.2 and Hap.3, the Hap.1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the yield traits of the rice to be tested are identified according to the following steps: homozygous rice to be tested having Hap. 2 as haplotype has lower yield, homozygous rice to be tested having Hap. 3 as haplotype has higher yield, and homozygous rice to be tested having Hap. 1 as a haplotype is not different from both the homozygous rice to be tested having Hap. 2 as haplotype and the homozygous rice to be tested having Hap. 3 as haplotype in yield.

19. A method for detecting yield traits of rice, comprising: detecting haplotypes of rice to be tested, the haplotypes of the rice being Hap.1, Hap.2 and Hap.3, the Hap. 1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the yield traits of the rice to be tested are identified according to the following steps: homozygous rice to be tested having Hap. 2 as haplotype has lower grain yield per plant than that of homozygous rice to be tested having Hap. 3 as haplotype, and homozygous rice to be tested having Hap. 1 as a haplotype is not different from the homozygous rice to be tested having Hap. 2 as haplotype and the homozygous rice to be tested having Hap. 3 as haplotype in the grain yield per plant.

20. A method for breeding rice, comprising: detecting nucleotides in rice genome DNA at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0), and selecting the rice having nucleotides, in sequence, A, A, G, C, A, G, G, C, T, and G, or G, G, A, G, T, A, A, T, G, and T at positions 1433007, 1433155, 1433229, 1433365, 1433528, 1433919, 1434216, 1434258, 1434486, and 1434806 as parent plants for breeding.

21. Use of a material for detecting rice haplotypes in detecting yield traits of rice, the haplotypes of the rice being Hap.1, Hap.2 and Hap.3, the Hap. 1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0).

22. Use of a material for detecting rice haplotypes in the preparation of a product for detecting yield traits of rice, the haplotypes of the rice being Hap.1, Hap.2 and Hap.3, the Hap. 1 having nucleotides, in sequence, A, A, G, C, A, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 2 having nucleotides, in sequence, A, G, G, C, T, G, G, C, T, and G at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0);
the Hap. 3 having nucleotides, in sequence, G, G, A, G, T, A, A, T, G, and T at physical positions 1433007, 1433155, 1433229, 1433365, 1433528, 1433919, 1434216, 1434258, 1434486, and 1434806 within the rice reference genome version number Os-Nipponbare-Reference-IRGSP-1.0 (IRGSP-1.0).
